(19) **Europäisches Patentamt**
**European Patent Office**
**Office européen des brevets**

(11) **EP 4 482 393 B1**

(12) **EUROPÄISCHE PATENTSCHRIFT**

(45) Veröffentlichungstag und Bekanntmachung des Hinweises auf die Patenterteilung:
**21.01.2026 Patentblatt 2026/04**

(21) Anmeldenummer: **23703492.1**

(22) Anmeldetag: **10.02.2023**

(51) Internationale Patentklassifikation (IPC):
*A61B 6/00* (2024.01)    *A61B 8/00* (2006.01)
*G16H 30/00* (2018.01)    *G16H 30/40* (2018.01)
*A61B 8/08* (2006.01)    *G01R 33/56* (2006.01)
*G06N 3/0455* (2023.01)    *G06N 3/084* (2023.01)
*G16H 50/20* (2018.01)    *G16H 50/70* (2018.01)
*G06N 3/0442* (2023.01)    *G06N 3/0464* (2023.01)
*G06N 3/02* (2006.01)    *A61B 6/03* (2006.01)

(52) Gemeinsame Patentklassifikation (CPC):
**A61B 6/481; A61B 8/481; G06N 3/0442;**
**G06N 3/0455; G06N 3/0464; G06N 3/084;**
**G16H 30/40; G16H 50/20; G16H 50/70;**
A61B 6/032; A61B 6/52; A61B 8/52; G01R 33/5601;
G01R 33/5608

(86) Internationale Anmeldenummer:
**PCT/EP2023/053324**

(87) Internationale Veröffentlichungsnummer:
**WO 2023/161041 (31.08.2023 Gazette 2023/35)**

(54) **VORHERSAGE VON REPRÄSENTATIONEN EINES UNTERSUCHUNGSBEREICHS EINES UNTERSUCHUNGSOBJEKTS NACH APPLIKATIONEN UNTERSCHIEDLICHER MENGEN EINES KONTRASTMITTELS**

PREDICTION OF A REPRESENTATION OF AN AREA OF AN OBJECT TO BE EXAMINED AFTER THE APPLICATION OF DIFFERENT AMOUNTS OF A CONTRAST AGENT

PRÉDICTION D'UNE REPRÉSENTATION D'UNE ZONE D'EXAMEN D'UN OBJET D'EXAMEN APRÈS L'APPLICATION DE DIFFÉRENTES QUANTITÉS D'UN AGENT DE CONTRASTE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **24.02.2022 EP 22158528**

(43) Veröffentlichungstag der Anmeldung:
**01.01.2025 Patentblatt 2025/01**

(73) Patentinhaber: **Bayer Aktiengesellschaft**
**51373 Leverkusen (DE)**

(72) Erfinder:
• **LENGA, Matthias**
**51373 Leverkusen (DE)**

• **SCHÜTZ, Gunnar**
**51373 Leverkusen (DE)**
• **VÖLKENING, Stephan**
**40789 Monheim am Rhein (DE)**

(74) Vertreter: **BIP Patents**
**c/o Bayer Intellectual Property GmbH**
**Alfred-Nobel-Straße 50**
**40789 Monheim am Rhein (DE)**

(56) Entgegenhaltungen:
**EP-A1- 3 739 522       WO-A1-2021/044153**
**US-A1- 2020 294 288**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft das technische Gebiet der Radiologie, insbesondere der Unterstützung von Radiologen bei radiologischen Untersuchungen mit Methoden der künstlichen Intelligenz. Die vorliegende Erfindung befasst sich mit dem Trainieren eines Modells des maschinellen Lernens und der Nutzung des trainierten Modells zur Vorhersage von Repräsentationen eines Untersuchungsbereichs nach Applikationen unterschiedlicher Mengen eines Kontrastmittels.

WO2021/044153A1 offenbart ein Verfahren und eine Vorrichtung zur Verbesserung von Magnetresonanzbildern, um kontrastverstärkte Bilder zu erzeugen, ohne dass einem Patienten ein Kontrastmittel verabreicht werden muss.

US2020294288A1 offenbart Verfahren zur Rekonstruktion eines Bildes eines kontrastverstärkten CT-Ausgangsbildes aus einem nicht verstärkten CT-Eingangsbild, zur Rekonstruktion eines nicht verstärkten CT-Ausgangsbildes aus einem kontrastverstärkten CT-Bild, zur Rekonstruktion eines kontrastverstärkten Dual-Energie-CT-Ausgangsbildes aus einem kontrastverstärkten CT-Einzelbild und zur Rekonstruktion eines kontrastverstärkten CT-Volldosisbildes aus einem kontrastverstärkten CT-Niederdosisbild.

EP3739522A1 offenbart Systeme zum Trainieren eines maschinellen Lernmodells für die Bildverarbeitung, ein System zur Bildverbesserung, ein Verfahren zum Trainieren eines maschinellen Lernmodells, eine Bildgebungsanordnung, ein Computerprogrammelement und ein computerlesbares Medium.

WO2019/074938A1 offenbart ein Verfahren zur Reduzierung der Menge an Kontrastmittel bei der Erzeugung von radiologischen Aufnahmen mit Hilfe eines künstlichen neuronalen Netzes.

In einem ersten Schritt wird ein Trainingsdatensatz erzeugt. Der Trainingsdatensatz umfasst für jede Person einer Vielzahl an Personen i) eine native radiologische Aufnahme (*zero-contrast image*), ii) eine radiologische Aufnahme nach der Applikation einer geringen Menge an Kontrastmittel (*low-contrast image*) und iii) eine radiologische Aufnahme nach der Applikation einer Standardmenge an Kontrastmittel (*full-contrast image*).

In einem zweiten Schritt wird ein künstliches neuronales Netz trainiert, für jede Person des Trainingsdatensatzes auf Basis der nativen Aufnahme und der Aufnahme nach Applikation einer geringen Menge an Kontrastmittel eine künstliche radiologische Aufnahme vorherzusagen, die einen Aufnahmebereich nach der Applikation der Standardmenge an Kontrastmittel zeigt. Die gemessene radiologische Aufnahme nach der Applikation einer Standardmenge an Kontrastmittel dient beim Training jeweils als Referenz (*ground truth*).

In einem dritten Schritt kann das trainierte künstliche neuronale Netz verwendet werden, für eine neue Person auf Basis einer nativen Aufnahme und einer radiologischen Aufnahme nach der Applikation einer geringen Menge an Kontrastmittel eine künstliche radiologische Aufnahme zu erzeugen, die den aufgenommenen Bereich so zeigt wie er aussehen würde, wenn eine Standardmenge an Kontrastmittel appliziert worden wäre.

Bei dem in WO2019/074938A1 offenbarten Verfahren wird ein künstliches neuronales Netzwerk trainiert, die native radiologische Aufnahme und die radiologische Aufnahme nach der Applikation einer geringen Menge an Kontrastmittel auf die radiologische Aufnahme nach der Applikation einer Standardmenge an Kontrastmittel abzubilden (engl. *mapping*). Das künstliche neuronale Netz wird aber nicht trainiert, einen zunehmenden Einfluss von Kontrastmittel auf eine radiologische Aufnahme zu lernen und kann damit nicht eingesetzt werden, um radiologische Aufnahmen zu erzeugen, die einen Untersuchungsbereichs nach der Applikation unterschiedlicher Mengen an Kontrastmittel zeigen.

Insbesondere ist das in WO2019/074938A1 beschriebene künstliche neuronale Netzwerk nicht geeignet, radiologische Aufnahmen zu erzeugen, die einen Untersuchungsbereich nach einer Applikation einer größeren Menge als der Standardmenge an Kontrastmittel zu zeigen.

Ferner sind im Fall des in WO2019/074938A1 beschriebenen Verfahrens zur Erzeugung einer radiologischen Aufnahme, die einen Untersuchungsbereich nach einer Applikation der Standardmenge eines Kontrastmittels zeigt, stets mindestens zwei radiologische Aufnahmen (eine native radiologische Aufnahme und eine radiologische Aufnahme nach der Applikation einer geringen Menge des Kontrastmittels) erforderlich.

Ausgehend vom beschriebenen Stand der Technik stellte sich die Aufgabe, ein Modell des maschinellen Lernens bereitzustellen, mit dem künstliche radiologische Aufnahmen erzeugt werden können, die einen Untersuchungsbereich nach der Applikation unterschiedlicher Mengen eines Kontrastmittels zeigen. Das Modell soll ferner prinzipiell in der Lage sein, künstliche radiologische Aufnahmen nach der Applikation unterschiedlicher Mengen an Kontrastmittel auf Basis einer einzigen messtechnisch erzeugten Aufnahme zu erzeugen.

Diese Aufgaben werden durch die Gegenstände der unabhängigen Patentansprüche gelöst. Bevorzugte Ausführungsformen finden sich in den abhängigen Patentansprüchen, der vorliegenden Beschreibung sowie in den Zeichnungen.

Ein erster Gegenstand der vorliegenden Erfindung ist ein computer-implementiertes Verfahren zum Trainieren und Verwenden eines Modells des maschinellen Lernens. Das Trainingsverfahren umfasst:

- Empfangen von Trainingsdaten,

- wobei die Trainingsdaten für eine Vielzahl von Untersuchungsobjekten Repräsentationen $^{T}R_1$ bis $^{T}R_n$ eines Untersuchungsbereichs umfassen, wobei $n$ eine ganze Zahl ist, die größer als zwei ist,
- wobei jede Repräsentation $^{T}R_i$ den Untersuchungsbereich nach Applikation einer Menge $a_i$ eines Kontrastmittels repräsentiert, wobei $i$ eine ganze Zahl im Bereich von 1 bis $n$ ist, wobei die Mengen $a_1$ bis $a_n$ eine Folge von vorzugsweise zunehmenden Mengen bilden,

- Trainieren des Modells des maschinellen Lernens,

- wobei das Modell trainiert wird, ausgehend von der Repräsentation $^{T}R_1$ nacheinander Repräsentationen $^{T}R_2^*$ bis $^{T}R_n^*$ zu erzeugen,

- wobei die Erzeugung der Repräsentation $^{T}R_2^*$ zumindest anteilig auf Basis der Repräsentation $^{T}R_1$ erfolgt und die Erzeugung jeder weiteren Repräsentation $^{T}R_k^*$ zumindest anteilig auf Basis der jeweils zuvor erzeugten Repräsentation $^{T}R_{k-1}^*$ erfolgt, wobei $k$ ein Index ist, der ganze Zahlen von 3 bis $n$ durchläuft, wobei die Repräsentation $^{T}R_k^*$ den Untersuchungsbereich nach Applikation der Menge $a_k$ des Kontrastmittels repräsentiert und die Repräsentation $^{T}R_{k-1}^*$ den Untersuchungsbereich nach Applikation der Menge $a_{k-1}$ des Kontrastmittels repräsentiert,

- wobei mittels einer Fehlerfunktion Abweichungen zwischen den erzeugten Repräsentationen $^{T}R_j^*$ und den Repräsentationen $^{T}R_j$ quantifiziert werden, wobei $j$ ein Index ist, der die Zahlen von 2 bis $n$ durchläuft,

- wobei die Abweichungen durch Modifizieren von Modellparametern des Modells des maschinellen Lernens in einem Optimierungsverfahren minimiert werden,

- Verwenden des Modells des maschinellen Lernens zur Vorhersage.

Ein weiterer Gegenstand der vorliegenden Erfindung ist ein computer-implementiertes Verfahren zur Vorhersage einer Repräsentation eines Untersuchungsbereichs eines Untersuchungsobjekts. Das Vorhersageverfahren umfasst:

- Empfangen einer Repräsentation $R_p$ des Untersuchungsbereichs,

- wobei die Repräsentation $R_p$ den Untersuchungsbereich ohne Kontrastmittel oder nach Applikation einer Menge $a_p$ eines Kontrastmittels repräsentiert, wobei $p$ eine ganze Zahl ist, die kleiner als $n$ ist, wobei $n$ eine ganze Zahl größer als 2 ist,

- Zuführen der Repräsentation $R_p$ einem trainierten Modell des maschinellen Lernens,

- wobei das Modell des maschinellen Lernens auf Basis von Trainingsdaten trainiert worden ist, ausgehend von einer ersten Repräsentation $^{T}R_1$ nacheinander eine Zahl $n-1$ von Repräsentationen $^{T}R_2^*$ bis $^{T}R_n^*$ zu erzeugen,

- wobei die erste Repräsentation $^{T}R_1$ den Untersuchungsbereich ohne Kontrastmittel oder nach Applikation einer ersten Menge $a_1$ des Kontrastmittels repräsentiert und jede erzeugte Repräsentation $^{T}R_j^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden,

- wobei die Erzeugung der Repräsentation $^{T}R_2^*$ zumindest anteilig auf Basis der Repräsentation $^{T}R_1$ erfolgt und die Erzeugung jeder weiteren Repräsentation $^{T}R_k^*$ zumindest anteilig auf Basis der jeweils zuvor erzeugten Repräsentation $^{T}R_{k-1}^*$ erfolgt, wobei $k$ ein Index ist, der ganze Zahlen von 3 bis $n$ durchläuft,

- Empfangen einer oder mehrerer Repräsentationen $R_{p+q}^*$ des Untersuchungsbereichs von dem Modell des maschinellen Lernens,

- wobei jede der einen oder der mehreren Repräsentationen $R_{p+q}^*$ den Untersuchungsbereich nach der Applikation der Menge $a_{p+q}$ des Kontrastmittels repräsentiert, wobei $q$ ein Index ist, der die Zahlen von 1 bis $m$ durchläuft, wobei $m$ eine ganze Zahl ist, die kleiner als $n-1$ ist oder gleich $n-1$ ist oder größer als $n-1$ ist,

- Ausgeben und/oder Speichern und/oder Übermitteln der einen oder der mehreren Repräsentationen $R_{p+q}^*$.

Ein weiterer Gegenstand der vorliegenden Erfindung ist ein Computersystem umfassend

- eine Eingabeeinheit,
- eine Steuer- und Recheneinheit und
- eine Ausgabeeinheit,

wobei die Steuer- und Recheneinheit konfiguriert ist,

- die Eingabeeinheit zu veranlassen, eine Repräsentation $R_p$ eines Untersuchungsbereichs eines Untersuchungsobjekts zu empfangen, wobei die Repräsentation $R_p$ den Untersuchungsbereich ohne Kontrastmittel oder nach einer Applikation einer Menge $a_p$ eines Kontrastmittels repräsentiert, wobei $p$ eine ganze Zahl ist, die kleiner als $n$ ist, wobei $n$ eine ganze Zahl größer als 2 ist,

- die Repräsentation $R_p$ einem trainierten Modell des maschinellen Lernens zuzuführen, wobei das Modell des maschinellen Lernens auf Basis von Trainingsdaten trainiert worden ist, ausgehend von einer ersten Repräsentation $^TR_1$ nacheinander eine Zahl $n\text{-}1$ von Repräsentationen $^TR_2^*$ bis $^TR_n^*$ zu erzeugen, wobei die erste Repräsentation $^TR_1$ den Untersuchungsbereich ohne Kontrastmittel oder nach Applikation einer ersten Menge $a_1$ des Kontrastmittels repräsentiert und jede erzeugte Repräsentation $^TR_j^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^TR_2^*$ zumindest anteilig auf Basis der Repräsentation $^TR_1$ erfolgt und die Erzeugung jeder weiteren Repräsentation $^TR_k^*$ zumindest anteilig auf Basis der jeweils zuvor erzeugten Repräsentation $^TR_{k\text{-}1}^*$ erfolgt, wobei $k$ ein Index ist, der ganze Zahlen von 3 bis $n$ durchläuft,

- von dem Modell des maschinellen Lernens eine oder mehrere Repräsentationen $R_{p+q}^*$ des Untersuchungsbereichs zu empfangen, wobei jede der einen oder der mehreren Repräsentationen $R_{p+q}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n\text{-}1$ ist oder gleich $n\text{-}1$ ist oder größer als $n\text{-}1$ ist,

- die Ausgabeeinheit zu veranlassen, die eine oder die mehreren Repräsentationen $R_{p+q}^*$ zu speichern und/oder auszugeben und/oder an ein separates Computersystem zu übermitteln.

Ein weiterer Gegenstand der vorliegenden Erfindung ist ein Computerprogrammprodukt umfassend einen Datenspeicher, in dem ein Computerprogramm gespeichert ist, das in einen Arbeitsspeicher eines Computersystems geladen werden kann und dort das Computersystem dazu veranlasst, folgende Schritte ausführen:

- Empfangen einer Repräsentation $R_p$ eines Untersuchungsbereichs eines Untersuchungsobjekts, wobei die Repräsentation $R_p$ den Untersuchungsbereich ohne Kontrastmittel oder nach Applikation einer Menge $a_p$ eines Kontrastmittels repräsentiert, wobei $p$ eine ganze Zahl ist, die kleiner als $n$ ist, wobei $n$ eine ganze Zahl größer als 2 ist,

- Zuführen der Repräsentation $R_p$ einem trainierten Modell des maschinellen Lernens, wobei das Modell des maschinellen Lernens auf Basis von Trainingsdaten trainiert worden ist, ausgehend von einer ersten Repräsentation $^TR_1$ nacheinander eine Zahl $n\text{-}1$ von Repräsentationen $^TR_2^*$ bis $^TR_n^*$ zu erzeugen, wobei die erste Repräsentation $^TR_1$ den Untersuchungsbereich ohne Kontrastmittel oder nach Applikation einer ersten Menge $a_1$ des Kontrastmittels repräsentiert und jede erzeugte Repräsentation $^TR_j^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^TR_2^*$ zumindest anteilig auf Basis der Repräsentation $^TR_1$ erfolgt und die Erzeugung jeder weiteren Repräsentation $^TR_k^*$ zumindest anteilig auf Basis der jeweils zuvor erzeugten Repräsentation $^TR_{k\text{-}1}^*$ erfolgt, wobei $k$ ein Index ist, der ganze Zahlen von 3 bis $n$ durchläuft,

- Empfangen einer oder mehrerer Repräsentationen $R_{p+q}^*$ des Untersuchungsbereichs von dem Modell des maschinellen Lernens, wobei jede der einen oder der mehreren Repräsentationen $R_{p+q}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n\text{-}1$ ist oder gleich $n\text{-}1$ ist oder größer als $n\text{-}1$ ist,

- Ausgeben und/oder Speichern und/oder Übermitteln der einen oder der mehreren Repräsentationen $R_{p+q}^*$.

Ein weiterer Gegenstand der vorliegenden Erfindung ist eine Verwendung eines Kontrastmittels in einem radiologischen Untersuchungsverfahren, wobei das radiologische Untersuchungsverfahren die folgenden Schritte umfasst:

- Empfangen einer Repräsentation $R_p$ eines Untersuchungsbereichs eines Untersuchungsobjekts, wobei die Repräsentation $R_p$ den Untersuchungsbereich ohne Kontrastmittel oder nach Applikation einer Menge $a_p$ des Kontrastmittels repräsentiert, wobei $p$ eine ganze Zahl ist, die kleiner als $n$ ist, wobei $n$ eine ganze Zahl größer als 2 ist,
- Zuführen der Repräsentation $R_p$ einem trainierten Modell des maschinellen Lernens, wobei das Modell des maschinellen Lernens auf Basis von Trainingsdaten trainiert worden ist, ausgehend von einer ersten Repräsentation $^{T}R_1$ nacheinander eine Zahl $n-1$ von Repräsentationen $^{T}R_2^*$ bis $^{T}R_n^*$ zu erzeugen, wobei die erste Repräsentation $^{T}R_1$ den Untersuchungsbereich ohne Kontrastmittel oder nach Applikation einer ersten Menge $a_1$ des Kontrastmittels repräsentiert und jede erzeugte Repräsentation $^{T}R_j^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^{T}R_2^*$ zumindest anteilig auf Basis der Repräsentation $^{T}R_1$ erfolgt und die Erzeugung jeder weiteren Repräsentation $^{T}R_k^*$ zumindest anteilig auf Basis der jeweils zuvor erzeugten Repräsentation $^{T}R_{k-1}^*$ erfolgt, wobei $k$ ein Index ist, der ganze Zahlen von 3 bis $n$ durchläuft,
- Empfangen einer oder mehrerer Repräsentationen $R_{p+q}^*$ des Untersuchungsbereichs von dem Modell des maschinellen Lernens, wobei jede der einen oder der mehreren Repräsentationen $R_{p+q}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n-1$ ist oder gleich $n-1$ ist oder größer als $n-1$ ist,
- Ausgeben und/oder Speichern und/oder Übermitteln der einen oder der mehreren Repräsentationen $R_{p+q}^*$.

Ein weiterer Gegenstand der vorliegenden Erfindung ist ein Kit umfassend ein Kontrastmittel und ein Computerprogrammprodukt, wobei das Computerprogrammprodukt ein Computerprogramm umfasst, das in einen Arbeitsspeicher eines Computersystems geladen werden kann und dort das Computersystem dazu veranlasst, folgende Schritte ausführen:

- Empfangen einer Repräsentation $R_p$ eines Untersuchungsbereichs eines Untersuchungsobjekts, wobei die Repräsentation $R_p$ den Untersuchungsbereich ohne Kontrastmittel oder nach Applikation einer Menge $a_p$ des Kontrastmittels repräsentiert, wobei $p$ eine ganze Zahl ist, die kleiner als $n$ ist, wobei $n$ eine ganze Zahl größer als 2 ist,
- Zuführen der Repräsentation $R_p$ einem trainierten Modell des maschinellen Lernens, wobei das Modell des maschinellen Lernens auf Basis von Trainingsdaten trainiert worden ist, ausgehend von einer ersten Repräsentation $^{T}R_1$ nacheinander eine Zahl $n-1$ von Repräsentationen $^{T}R_2^*$ bis $^{T}R_n^*$ zu erzeugen, wobei die erste Repräsentation $^{T}R_1$ den Untersuchungsbereich ohne Kontrastmittel oder nach Applikation einer ersten Menge $a_1$ des Kontrastmittels repräsentiert und jede erzeugte Repräsentation $^{T}R_j^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^{T}R_2^*$ zumindest anteilig auf Basis der Repräsentation $^{T}R_1$ erfolgt und die Erzeugung jeder weiteren Repräsentation $^{T}R_k^*$ zumindest anteilig auf Basis der jeweils zuvor erzeugten Repräsentation $^{T}R_{k-1}^*$ erfolgt, wobei $k$ ein Index ist, der ganze Zahlen von 3 bis $n$ durchläuft,
- Empfangen einer oder mehrerer Repräsentationen $R_{p+q}^*$ des Untersuchungsbereichs von dem Modell des maschinellen Lernens, wobei jede der einen oder der mehreren Repräsentationen $R_{p+q}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n-1$ ist oder gleich $n-1$ ist oder größer als $n-1$ ist,
- Ausgeben und/oder Speichern und/oder Übermitteln der einen oder der mehreren Repräsentationen $R_{p+q}^*$.

Die Erfindung wird nachstehend näher erläutert, ohne zwischen den Erfindungsgegenständen (Trainingsverfahren, Vorhersageverfahren, Computersystem, Computerprogrammprodukt, Verwendung, Kit) zu unterscheiden. Die nachfolgenden Erläuterungen sollen vielmehr für alle Erfindungsgegenstände in analoger Weise gelten, unabhängig davon, in welchem Kontext (Trainingsverfahren, Vorhersageverfahren, Computersystem, Computerprogrammprodukt, Verwendung, Kit) sie erfolgen. Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar

Mit Hilfe der vorliegenden Erfindung können Repräsentationen eines Untersuchungsbereichs eines Untersuchungsobjekts vorhergesagt werden.

Das "Untersuchungsobjekt" ist üblicherweise ein Lebewesen, vorzugsweise ein Säugetier, ganz besonders bevorzugt ein Mensch.

Der "Untersuchungsbereich" ist ein Teil des Untersuchungsobjekts, zum Beispiel ein Organ oder ein Teil eines Organs wie beispielsweise die Leber, das Gehirn, das Herz, die Niere, die Lunge, der Magen, der Darm, oder die Blase oder ein Teil der genannten Organe oder mehrere Organe oder ein anderer Teil des Körpers.

In einer bevorzugten Ausführungsform der vorliegenden Erfindung ist der Untersuchungsbereich die Leber oder ein Teil der Leber eines Menschen.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

Eine "Repräsentation des Untersuchungsbereichs" ist vorzugsweise eine medizinische Aufnahme (engl.: *medical image).* Eine "Repräsentation des Untersuchungsbereichs" ist vorzugsweise das Ergebnis einer radiologischen Untersuchung.

Die "Radiologie" ist das Teilgebiet der Medizin, das sich mit der Anwendung vorwiegend elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlungen wie Gammastrahlung oder Elektronen zum Einsatz. Da ein wesentlicher Einsatzzweck die Bildgebung ist, werden auch andere bildgebende Verfahren wie die Sonografie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Erfindung umfasst damit insbesondere die folgenden Untersuchungsmethoden: Computertomografie, Magnetresonanztomografie, Sonografie.

In einer bevorzugten Ausführungsform der vorliegenden Erfindung handelt es sich bei der radiologischen Untersuchung um eine computertomographische Untersuchung oder eine magnetresonanztomographische Untersuchung.

Computertomographie (CT) ist ein Röntgenverfahren, mit dem der menschliche Körper in Querschnittbildern (Schnittbildverfahren) dargestellt wird. Im Vergleich zu einer herkömmlichen Röntgenaufnahme, auf der üblicherweise nur grobe Strukturen und Knochen erkennbar sind, wird in CT-Aufnahmen auch Weichteilgewebe mit geringen Kontrastunterschieden detailliert erfasst. Eine Röntgenröhre erzeugt einen sogenannten Röntgenfächerstrahl, der den Körper durchdringt und innerhalb des Körpers durch die verschiedenen Strukturen, wie Organe und Knochen, unterschiedlich stark abgeschwächt wird. Die Empfangsdetektoren gegenüber dem Röntgenstrahler empfangen die unterschiedlich starken Signale und leiten sie an einen Computer weiter, der aus den empfangenen Daten Schichtbilder des Körpers zusammensetzt. Computertomografische Aufnahmen (CT-Aufnahmen) können in 2D oder auch in 3D betrachtet werden. Zur besseren Abgrenzbarkeit von Strukturen innerhalb des Körpers des Menschen (z.B. Gefäße), kann vor der Erzeugung von CT-Aufnahmen ein Kontrastmittel z.B. in eine Vene gespritzt werden.

Die Magnetresonanztomographie, abgekürzt MRT oder MRI (engl. MRI: *Magnetic Resonance Imaging),* ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten MR-Daten liegen zunächst als Rohdaten im Frequenzraum vor, und können durch anschließende inverse Fourier-Transformation in den Ortsraum (Bildraum) transformiert werden.

Auch in der Magnetresonanztomographie können Kontrastmittel eingesetzt werden, um eine Kontrastverstärkung zu erreichen.

"Kontrastmittel" sind Substanzen oder Gemische von Substanzen, die die Darstellung von Strukturen und Funktionen des Körpers bei bildgebenden radiologischen Verfahren verbessern.

Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wp-content/uploads/2017/10/contrast-agents-tutorial.pdf, M. R. Nough et al.: Radiographic and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review of Risk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

Eine Repräsentation des Untersuchungsbereichs im Sinne der vorliegenden Erfindung kann ein Computertomogramm, eine MRT-Aufnahme, ein Ultraschallbild oder dergleichen sein.

Eine Repräsentation des Untersuchungsbereichs im Sinne der vorliegenden Erfindung kann eine Repräsentation im Ortsraum (Bildraum) oder eine Repräsentation im Frequenzraum oder eine andere Repräsentation sein. Vorzugsweise liegen Repräsentationen des Untersuchungsbereichs in einer Ortsraum-Darstellung oder in einer Form vor, die sich in eine Ortsraum-Darstellung umwandeln (transformieren) lässt. Eine Repräsentation im Ortsraum wird oftmals auch als bildhafte Darstellung oder als Bild (engl.: *image)* bezeichnet.

In einer Repräsentation im Ortsraum, in dieser Beschreibung auch als Ortsraum-Darstellung oder Ortsraum-Repräsentation bezeichnet, wird der Untersuchungsbereich üblicherweise durch eine Vielzahl an Bildelementen (Pixeln oder Voxel) repräsentiert, die beispielsweise rasterförmig angeordnet sein können, wobei jedes Bildelement einen Teil des Untersuchungsbereichs repräsentiert, wobei jedem Bildelement ein Farbwert oder Grauwert zugeordnet sein kann. Ein in der Radiologie weit verbreitetes Format zur Speicherung und Verarbeitung von Repräsentationen im Ortsraum ist das DICOM-Format. DICOM (Digital Imaging and Communications in Medicine) ist ein offener Standard zur Speicherung und zum Austausch von Informationen im medizinischen Bilddatenmanagement.

Eine Repräsentation im Ortsraum lässt sich beispielsweise durch eine Fourier-Transformation in eine Repräsentation im Frequenzraum überführen (transformieren). Umgekehrt lässt sich eine Repräsentation im Frequenzraum beispielsweise durch eine inverse Fourier-Transformation in eine Repräsentation im Ortsraum überführen (transformieren).

In einer Repräsentation im Frequenzraum, in dieser Beschreibung auch als Frequenzraum-Darstellung oder Frequenzraum-Repräsentation bezeichnet, wird der Untersuchungsbereich durch eine Überlagerung von Grundschwingungen repräsentiert. Beispielsweise kann der Untersuchungsbereich durch eine Summe von Sinus- und/oder Kosinus-Funktionen mit verschiedenen Amplituden, Frequenzen und Phasen repräsentiert werden. Die Amplituden und Phasen können als Funktion der Frequenzen beispielsweise in einer zwei- oder dreidimensionalen Darstellung aufgetragen werden. Üblicherweise wird die niedrigste Frequenz (Ursprung) in das Zentrum gelegt. Je weiter man sich von diesem Zentrum entfernt, desto höher sind die Frequenzen. Jeder Frequenz kann eine Amplitude, mit der die Frequenz in der Frequenzraumdarstellung vertreten ist, und eine Phase, die angibt, inwieweit die jeweilige Schwingung gegenüber einer Sinus- oder Kosinus-Schwingung verschoben ist, zugeordnet werden.

Details über Ortsraum-Darstellungen und Frequenzraum-Darstellungen und ihre jeweilige Umwandlung ineinander sind in zahlreichen Publikationen beschrieben, siehe z.B.: https://see.stanford.edu/materials/lsoftaee261/book-fall-07.pdf.

Eine Repräsentation im Sinne der vorliegenden Erfindung repräsentiert den Untersuchungsbereich vor oder nach der Applikation einer Menge eines Kontrastmittels.

Unter dem Begriff "Menge" kann die absolute Menge, die einem Untersuchungsobjekt verabreicht wurde, verstanden werden (z.B. gemessen in Kilogramm oder Mol oder Liter); es kann sich bei der "Menge" aber auch um eine verabreichte Dosis handeln, wie beispielsweise eine Menge an Kontrastmittel (z.B. gemessen in Kilogramm oder Mol oder Liter) pro Kilogramm Körpergewicht des Untersuchungsobjekts. Es kann sich bei dem Begriff "Menge" aber auch um eine Konzentration handeln, die sich zumindest für einen definierten Zeitpunkt nach der Applikation des Kontrastmittels in dem Untersuchungsobjekt einstellt. Weitere Mengen-Definitionen sind denkbar.

Vorzugsweise handelt es sich bei dem Kontrastmittel um ein MR-Kontrastmittel. MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide*). Beispiele für paramagnetische Kontrastmittel sind Gadolinium-Chelate wie Gadopentetat-Dimeglumin (Handelsname: Magnevist® u.a.), Gadotersäure (Dotarem®, Dotagita®, Cyclolux®), Gadodiamid (Omniscan®), Gadoteridol (ProHance®) und Gadobutrol (Gadovist®).

Vorzugsweise handelt es sich bei dem MR-Kontrastmittel um ein hepatobiliäres Kontrastmittel. Ein hepatobiliäres Kontrastmittel zeichnet sich dadurch aus, dass es spezifisch von Leberzellen, den Hepatozyten, aufgenommen wird, sich im Funktionsgewebe (Parenchym) anreichert und den Kontrast in gesundem Lebergewebe verstärkt. Ein Beispiel eines hepatobiliären Kontrastmittels ist das Dinatriumsalz der Gadoxetsäure (Gd-EOB-DTPA Dinatrium), das in dem US-Patent No. 6,039,931A beschrieben ist unter dem Markennamen Primovist® und Eovist® kommerziell erhältlich ist.

Das Kontrastmittel kann gewichtsadaptiert in Form einer Bolusinjektion beispielsweise in eine Armvene appliziert werden.

Mit Hilfe der vorliegenden Erfindung können Repräsentationen eines Untersuchungsbereichs nach der Applikation unterschiedlicher Mengen eines Kontrastmittels vorhergesagt werden. Dabei kann die Vorhersage prinzipiell auf Basis einer einzigen Repräsentation erfolgen, die den Untersuchungsbereich ohne Kontrastmittel oder nach der Applikation einer ersten Menge eines Kontrastmittels repräsentiert.

Die Vorhersage erfolgt mit Hilfe eines Modells des maschinellen Lernens.

Ein "Modell des maschinellen Lernens" kann als eine computerimplementierte Datenverarbeitungsarchitektur verstanden werden. Das Modell kann Eingabedaten empfangen und Ausgabedaten auf der Grundlage dieser Eingabedaten und Modell-Parametern liefern. Das Modell kann durch Training eine Beziehung zwischen den Eingabedaten und den Ausgabedaten erlernen. Beim Training können Modell-Parameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modell-Parameter werden so verändert, dass die Abweichungen zwischen den Ausgabedaten und den Zieldaten auf ein (definiertes) Minimum reduziert werden.

Die Abweichungen können mit Hilfe einer Fehlerfunktion (engl.: *loss function*) quantifiziert werden. Eine solche Fehlerfunktion kann verwendet werden, um einen Fehlerwert (engl.: *loss value*) für ein gegebenes Paar von Ausgabedaten und Zieldaten zu berechnen. Das Ziel des Trainingsprozesses kann darin bestehen, die Parameter des maschinellen Lernmodells so zu verändern (anzupassen), dass der Fehlerwert für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehlerwert bedeuten, dass ein oder mehrere Modell-Parameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder jede andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Im vorliegenden Fall wird das Modell des maschinellen Lernens anhand von Trainingsdaten trainiert, ausgehend von einer ersten Repräsentation, die einen Untersuchungsbereich ohne Kontrastmittel oder nach der Applikation einer ersten Menge eines Kontrastmittels repräsentiert, eine Folge von Repräsentationen vorherzusagen, die den Untersuchungsbereich nach der Applikation unterschiedlicher Mengen des Kontrastmittels repräsentieren.

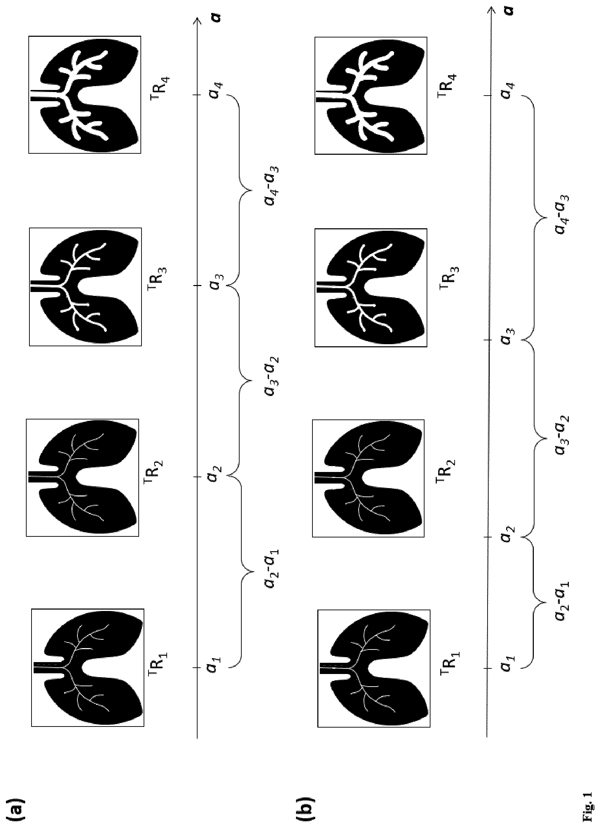

Die unterschiedlichen Mengen an Kontrastmittel bilden eine Folge, in der die Kontrastmittelmengen beispielsweise zunehmen oder abnehmen können. Dies sei anhand von Fig. 1 erläutert.

Fig. 1 zeigt beispielhaft und schematisch vier Ortsraum-Repräsentationen eines Untersuchungsbereichs nach der Applikation unterschiedlicher Mengen eines Kontrastmittels. Bei dem Untersuchungsbereich handelt es sich um die Lunge eines Menschen. Die vier Repräsentationen $^T R_1$, $^T R_2$, $^T R_3$ und $^T R_4$ sind entlang einer Achse aufgereiht, wobei die Achse die Menge $a$ an Kontrastmittel, die dem Untersuchungsobjekt jeweils verabreicht worden ist, angibt. Die Repräsentation $^T R_1$ zeigt also den Untersuchungsbereich nach einer Applikation einer ersten Menge $a_1$ des Kontrastmittels; die Repräsentation $^T R_2$ zeigt den Untersuchungsbereich nach einer Applikation einer zweiten Menge $a_2$ des Kontrastmittels; die Repräsentation $^T R_3$ zeigt den Untersuchungsbereich nach einer Applikation einer dritten Menge $a_3$ des Kontrastmittels; die Repräsentation $^T R_4$ zeigt den Untersuchungsbereich nach einer Applikation einer vierten Menge $a_4$ des Kontrastmittels. Die erste Menge $a_1$ kann größer oder gleich Null sein, die zweite Menge $a_2$, die dritte Menge $a_3$ und die vierte Menge $a_4$ sind in dem vorliegenden Beispiel jeweils größer als Null. Im vorliegenden Beispiel ist die zweite Menge $a_2$ größer als die erste Menge $a_1$, die dritte Menge $a_3$ größer als die zweite Menge $a_2$ und die vierte Menge $a_4$ größer als die dritte Menge $a_3$: $0 \leq a_1 < a_2 < a_3 < a_4$

In dem in Fig. 1 gezeigten Beispiel bilden die Kontrastmittelmengen also eine Folge zunehmender Kontrastmittelmengen.

Dies zeigt sich in Fig. 1 in den Repräsentationen $^T R_1$ bis $^T R_4$ durch eine zunehmende Kontrastverstärkung der Blutgefäße gegenüber dem umliegenden Gewebe.

In dem in Fig. 1 (a) gezeigten Beispiel nimmt die Menge um einen gleichbleibenden Betrag zu, d.h. die Differenz der Mengen zweier unmittelbar aufeinanderfolgender Mengen ist für alle unmittelbar aufeinanderfolgenden Mengen gleich: $a_2 - a_1 = a_3 - a_2 = a_4 - a_3$

In einer Folge von Kontrastmittelmengen können die Mengen jedoch auch um einen unterschiedlichen Betrag zunehmen.

In dem in Fig. 1 (b) gezeigten Beispiel nimmt die Menge um einen zunehmenden Betrag zu: $a_2 - a_1 < a_3 - a_2 < a_4 - a_3$

Es ist aber auch möglich, dass die Mengen um einen abnehmenden Betrag zunehmen: $a_2 - a_1 > a_3 - a_2 > a_4 - a_3$ Es ist aber auch möglich, dass die Repräsentationen anders entlang der Mengen-Achse verteilt sind.

Ferner ist es möglich, dass die Kontrastmittelmengen eine Folge abnehmender Mengen bildet oder eine andere Folge bildet.

Es sei darauf hingewiesen, dass die unterschiedlichen Mengen an Kontrastmittel üblicherweise nicht unmittelbar aufeinanderfolgend appliziert werden. Eine additive Wirkung der unterschiedlichen Mengen soll üblicherweise verhindert werden. Um für die Trainingsdaten einen Datensatz für ein Untersuchungsobjekt zu erzeugen, der zwei oder mehr Repräsentationen eines Untersuchungsbereichs nach der Applikation unterschiedlicher Mengen eines Kontrastmittels repräsentiert, werden die unterschiedlichen Mengen üblicherweise in einem zeitlichen Abstand zueinander appliziert, der so groß ist, dass die in dem Untersuchungsbereich vorhandene Menge an Kontrastmittel aus einer früheren Applikation zum Zeitpunkt einer späteren Applikation einer anderen Menge an Kontrastmittel so gering ist, dass sie auf die Repräsentation des Untersuchungsbereichs keinen messbaren Einfluss ausübt. Mit anderen Worten, der Ausdruck, "die Repräsentation repräsentiert den Untersuchungsbereich nach einer Menge eines Kontrastmittels" bedeutet vorzugsweise "die Repräsentation repräsentiert den Untersuchungsbereich mit einer Menge eines Kontrastmittels".

Es sei darauf hingewiesen, dass Repräsentationen des Untersuchungsbereichs in dieser Beschreibung mit dem Buchstaben R gekennzeichnet sind. Dem Buchstaben R kann ein Index nachgestellt sein, wie beispielsweise $R_1$, $R_2$, $R_3$ oder $R_i$. Dabei gibt der nachgestellte Index an, was die jeweilige Repräsentation repräsentiert. Die Repräsentation $R_1$ repräsentiert den Untersuchungsbereich eines Untersuchungsobjekts nach der Applikation der Menge $a_1$ eines Kontrastmittels; die Repräsentation $R_2$ repräsentiert den Untersuchungsbereich eines Untersuchungsobjekts nach der Applikation der Menge $a_2$ eines Kontrastmittels; die Repräsentation $R_3$ repräsentiert den Untersuchungsbereich eines Untersuchungsobjekts nach der Applikation der Menge $a_3$ eines Kontrastmittel, und so fort. Allgemein repräsentiert die Repräsentation $R_i$ den Untersuchungsbereich eines Untersuchungsobjekts nach der Applikation der Menge $a_i$ eines Kontrastmittel, wobei $i$ ein Index ist, der die ganzen Zahlen von $i$ bis $n$ durchläuft, wobei $n$ eine ganze Zahl größer als zwei ist. Der Ausdruck "ein Index $i$ durchläuft die ganzen Zahlen von $a$ bis $b$" bedeutet, dass $i$ nacheinander die Werte von $a$ bis $b$ annimmt, also zunächst den Wert $a$ $(i=a)$, dann den Wert $a+1$ $(i=a+1)$ und so weiter, bis $i$ den Wert b erreicht $(i=b)$. Der Ausdruck "jede erzeugte Repräsentation $R_j^*$ repräsentiert den Untersuchungsbereich nach Applikation einer Menge $a_j$ des Kontrastmittels" bedeutet, dass die Repräsentation $R_1^*$ den Untersuchungsbereich nach Applikation einer Menge $a_1$ des Kontrastmittels repräsentiert, die Repräsentation $R_2^*$ den Untersuchungsbereich nach Applikation einer Menge $a_2$ des Kontrastmittels repräsentiert, und so fort. Repräsentationen, die zum Training verwendet werden, und Repräsentationen, die im Training erzeugt werden, werden insbesondere in den Patentansprüchen und den Zeichnungen mit einem vorangestellten $^T$ gekennzeichnet, wie beispielsweise im Fall von $^TR_1$, $^TR_2$ und $^TR_{k-1}^*$. Mittels des Modells des maschinellen Lernens erzeugte Repräsentationen werden insbesondere in den Patentansprüchen und den Zeichnungen durch ein hochgestelltes Stern-Symbol * gekennzeichnet, wie im Fall von $R_1^*$, $R_2^*$ und $^TR_{k-1}^*$. Die hier beschriebenen Kennzeichnungen dienen lediglich der Klarstellung, insbesondere der Vermeidung von Klarheitseinwänden im Patenterteilungsverfahren.

Das Modell des maschinellen Lernens kann anhand von Trainingsdaten trainiert werden, ausgehend von der ersten Repräsentation $^TR_1$ nacheinander die Repräsentationen $^TR_2$, $^TR_3$ und $^TR_4$ vorherzusagen. Mit anderen Worten: das Modell des maschinellen Lernens kann die Folge von Kontrastmittelmengen lernen, um sie dann auf neue Repräsentationen anzuwenden.

Das Training wird nachfolgend der Einfachheit halber zunächst auf Basis von drei Repräsentationen erläutert. Anschließend erfolgt eine Erweiterung auf eine beliebige Zahl $n$ an Repräsentationen, wobei $n$ eine ganze Zahl größer als zwei ist.

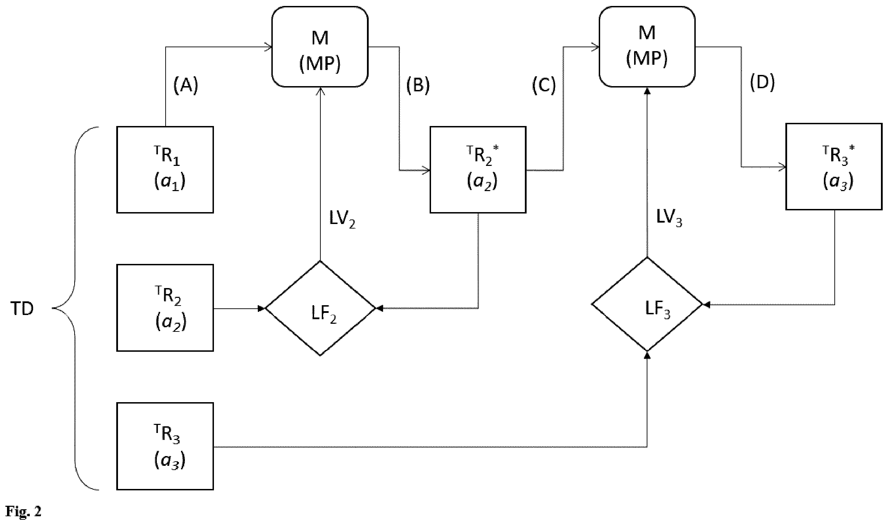

Bei der nachfolgenden Erläuterung des Trainings auf Basis von drei Repräsentationen wird auf Fig. 2 Bezug genommen.

Fig. 2 zeigt drei Repräsentationen, eine erste Repräsentation $^TR_1$, eine zweite Repräsentation $^TR_2$ und eine dritte Repräsentation $^TR_3$.

Die erste Repräsentation $^TR_1$ repräsentiert einen Untersuchungsbereich eines Untersuchungsobjekts ohne Kontrastmittel oder nach einer Applikation einer ersten Menge $a_1$ eines Kontrastmittels, die zweite Repräsentation $^TR_2$ repräsentiert den Untersuchungsbereich nach einer Applikation einer zweiten Menge $a_2$ des Kontrastmittels und die dritte Repräsentation $^TR_3$ repräsentiert den Untersuchungsbereich nach einer Applikation einer dritten Menge $a_3$ des Kontrastmittels. Die Kontrastmittelmengen $a_1$, $a_2$ und $a_3$ können beispielsweise eine Folge zunehmender Mengen an Kontrastmittel bilden ($0 \leq a_1 < a_2 < a_3$).

Die drei Repräsentationen $^TR_1$, $^TR_2$ und $^TR_3$ bilden einen Datensatz innerhalb von Trainingsdaten TD. Die Trainingsdaten TD umfassen eine Vielzahl solcher Datensätze. Der Begriff Vielzahl bedeutet vorzugsweise mehr als 100. Jeder Datensatz umfasst mindestens zwei, üblicherweise drei oder mehr Repräsentationen des Untersuchungsbereichs nach der Applikation der jeweiligen Mengen des Kontrastmittels (die im Fall von $a_1$ auch Null sein kann). Dabei ist der Untersuchungsbereich bei allen Repräsentationen der gleiche; lediglich das Untersuchungsobjekt kann variieren; üblicherweise stammt jeder Datensatz von einem anderen Untersuchungsobjekt. Die in diesem Absatz getätigten Aussagen gelten allgemein und nicht nur in Bezug auf das in Fig. 2 gezeigte Beispiel.

In einem ersten Schritt (A) wird die erste Repräsentation $^TR_1$ einem Modell M des maschinellen Lernens zugeführt. Das Modell M des maschinellen Lernens ist konfiguriert, auf Basis der ersten Repräsentation $^TR_1$ und auf Basis von Modellparametern MP eine Ausgabe $^TR_2^*$ zu erzeugen (Schritt (B)).

Die Ausgabe $^TR_2^*$ soll der zweiten Repräsentation $^TR_2$ möglichst nahekommen; im Idealfall kann die Ausgabe $^TR_2^*$ nicht von der zweiten Repräsentation $^TR_2$ unterschieden werden. Mit anderen Worten: bei der Ausgabe $^TR_2^*$ handelt es sich um eine vorhergesagte zweite Repräsentation. Die Ausgabe $^TR_2^*$ wird mit der (realen) zweiten Repräsentation $^TR_2$ verglichen und die Abweichungen werden mit Hilfe einer Fehlerfunktion $LF_2$ quantifiziert. Im Falle von mehreren Untersuchungsobjekten kann für jedes Paar von Ausgabe $^TR_2^*$ und zweiter Repräsentation $^TR_2$ ein Fehlerwert $LV_2$ mittels der Fehlerfunktion $LF_2$ berechnet werden.

Beispiele für Fehlerfunktionen, die allgemein (nicht beschränkt auf das Beispiel in Fig. 2) zur Ausführung der vorliegenden Erfindung verwendet werden können, sind L1-Fehlerfunktion (*L1 loss),* L2-Fehlerfunktion *(L2 loss),* Lp-Fehlerfunktion (Lp loss), Strukturähnlichkeitsindexmaß (*structural similarity index measure* (SSIM)), VGG-Fehlerfunktion (VGG loss), Wahrnehmungs-Fehlerfunktion (perceptual loss) oder eine Kombination der oben genannten Funktionen. Weitere Einzelheiten über Fehlerfunktionen sind z.B. in der wissenschaftlichen Literatur zu finden (siehe z. B.: R. Mechrez et al.: The Contextual Loss for Image Transformation with Non-Aligned Data, 2018, arXiv:1803.02077v4; H. Zhao et al.: Loss Functions for Image Restoration with Neural Networks, 2018, arXiv:1511.08861v3).

Die Ausgabe $^TR_2^*$ wird in Schritt (C) dem Modell M des maschinellen Lernens zugeführt. Auch wenn Fig. 2 einen anderen Eindruck erwecken mag: bei dem Modell M des maschinellen Lernens, dem in Schritt (A) die erste Repräsentation $^TR_1$ und in Schritt (C) die erzeugte Repräsentation $^TR_2^*$ zugeführt werden, handelt es sich um dasselbe Modell. Das heißt, das Modell M des maschinellen Lernens ist nicht nur konfiguriert, auf Basis der ersten Repräsentation $^TR_1$ eine Repräsentation $^TR_2^*$ zu erzeugen, sondern es ist auch konfiguriert, aus der Repräsentation $^TR_2^*$ eine Ausgabe $^TR_3^*$ zu erzeugen, die der dritten Repräsentation $^TR_3$ möglichst nahekommt. Die Ausgabe $^TR_3^*$ ist eine vorhergesagte dritte Repräsentation. Die Ausgabe $^TR_3^*$ wird mit der dritten Repräsentation $^TR_3$ verglichen. Mit Hilfe einer Fehlerfunktion $LF_3$ kann die Abweichung zwischen der Ausgabe $^TR_3^*$ und der dritten Repräsentation $^TR_3$ quantifiziert werden; es kann im Falle von mehreren Untersuchungsobjekten für jedes Paar einer dritten Repräsentation $^TR_3$ und einer erzeugten Repräsentation $^TR_3^*$ ein Fehlerwert $LV_3$ ermittelt werden.

Vorzugsweise wird das Modell des maschinellen Lernens Ende-zu-Ende (engl. *end-to-end*) trainiert. Das bedeutet, dass das Modell des maschinellen Lernens gleichzeitig trainiert wird, eine vorhergesagte zweite Repräsentation auf Basis der ersten Repräsentation und eine vorhergesagte dritte Repräsentation auf Basis der vorhergesagten zweiten Repräsentation zu erzeugen. Vorzugsweise wird dazu eine Fehlerfunktion verwendet, die sowohl die Abweichungen zwischen der vorhergesagten zweiten Repräsentation und der zweiten Repräsentation als auch die Abweichungen zwischen der vorhergesagten dritten Repräsentation und der dritten Repräsentation berücksichtigt.

Es ist möglich, die Abweichungen zwischen der Repräsentation $^TR_2$ und der erzeugten Repräsentation $^TR_2^*$ mit Hilfe der Fehlerfunktion $LF_2$ zu quantifizieren und die Abweichungen zwischen der dritten Repräsentation $^TR_3$ und der erzeugten Repräsentation $^TR_3^*$ mit Hilfe der Fehlerfunktion $LF_3$ zu quantifizieren. Eine Fehlerfunktion LF, die beide Abweichungen berücksichtigt, kann beispielsweise die Summe der einzelnen Fehlerfunktionen sein: $LF = LF_2 + LF_3$. Es ist auch möglich die Anteile, die die einzelnen Fehlerfunktionen $LF_2$ und $LF_3$ an der Gesamtfehlerfunktion LF haben, unterschiedlich zu gewichten: $LF = w_2 \cdot LF_2 + w_3 \cdot LF_3$, wobei $w_2$ und $w_3$ Gewichtsfaktoren sind, die beispielsweise Werte zwischen 0 und 1 annehmen können. Dabei kann der Wert Null für einen Gewichtsfaktor beispielsweise dann verwendet werden, wenn in einem Datensatz eine Repräsentation fehlt (mehr dazu ist in der Beschreibung weiter hinten zu finden). Die Gewichtsfaktoren können ferner während des Trainings verändert werden.

Das in Fig. 2 dargestellte Trainingsverfahren ist eine bevorzugte Ausführungsform der vorliegenden Offenbarung und umfasst die Schritte:

- Empfangen von Trainingsdaten,

   • wobei die Trainingsdaten für eine Vielzahl von Untersuchungsobjekten Repräsentationen $^TR_1$ bis $^TR_3$ eines Untersuchungsbereichs umfassen,

   • wobei die Repräsentation $^TR_1$ den Untersuchungsbereich ohne Kontrastmittel oder nach Applikation einer Menge $a_1$ eines Kontrastmittels repräsentiert, die Repräsentation $^TR_2$ den Untersuchungsbereich nach Applikation einer Menge $a_2$ des Kontrastmittels repräsentiert und die Repräsentation $^TR_3$ den Untersuchungsbereich nach Applikation einer Menge $a_3$ des Kontrastmittels repräsentiert, wobei die Mengen $a_1$ bis $a_3$ eine Folge von vorzugsweise zunehmenden Mengen bilden,

- Trainieren eines Modells des maschinellen Lernens, wobei das Trainieren für jedes Untersuchungsobjekt der Vielzahl von Untersuchungsobjekten umfasst:

  • Zuführen der Repräsentation $^TR_1$ dem Modell des maschinellen Lernens, wobei das Modell des maschinellen Lernens konfiguriert ist, zumindest anteilig auf Basis der Repräsentation $^TR_1$ und auf Basis von Modellparametern eine Repräsentation $^TR_2^*$ zu erzeugen,

  • Zuführen der Repräsentation $^TR_2^*$ dem Modell des maschinellen Lernens, wobei das Modell des maschinellen Lernens ferner konfiguriert ist, zumindest anteilig auf Basis der Repräsentation $^TR_2^*$ und auf Basis von Modellparametern eine Repräsentation $^TR_3^*$ zu erzeugen,

  • Quantifizieren von Abweichungen zwischen der Repräsentation $^TR_2$ und der erzeugten Repräsentation $^TR_2^*$ sowie zwischen der Repräsentation $^TR_3$ und der erzeugten Repräsentation $^TR_3^*$,

  • Minimieren der Abweichungen durch Modifizieren der Modellparameter,

- Speichern des trainierten Modells des maschinellen Lernens und/oder Verwenden des Modells des maschinellen Lernens zur Vorhersage.

Bei der (späteren) Verwendung des trainierten Modells des maschinellen Lernens zur Vorhersage kann es darum gehen, eine dritte Repräsentation auf Basis einer ersten Repräsentation vorherzusagen. Prinzipiell könnte für diesen Zweck ein Modell trainiert werden, die dritte Repräsentation direkt auf Basis der ersten Repräsentation zu erzeugen ("direktes Training"). Erfindungsgemäß wird das Modell des maschinellen Lernens jedoch trainiert, die dritte Repräsentation nicht direkt (in einem Schritt) auf Basis der ersten Repräsentation zu erzeugen, sondern in zwei Schritten, wobei in einem ersten Schritt eine zweite Repräsentation vorhergesagt wird, auf deren Basis dann die dritte Repräsentation vorhergesagt wird. Der Vorteil bei dem erfindungsgemäßen iterativen Ansatz gegenüber dem genannten "direkten Trainieren" besteht unter anderem darin, dass zusätzliche Trainingsdaten (z.B. zweite Repräsentationen, die den Untersuchungsbereich in dem zweiten Zustand repräsentieren), verwendet werden können und damit eine höhere Vorhersagegenauigkeit erreicht werden kann. Ferner lernt das Modell keine Abbildung von einer Repräsentation auf eine andere (oder, wie im Fall von WO2019/074938A1, eine Abbildung von mehreren Repräsentationen auf eine Repräsentation), sondern es lernt, welchen Einfluss Kontrastmittel auf die Repräsentationen des Untersuchungsbereichs ausübt, insbesondere wie sich zunehmende oder abnehmende Mengen an Kontrastmittel auf die Repräsentationen auswirken. Je mehr Mengen an Kontrastmittel die Trainingsdaten abdecken, desto genauer kann das Modell den Einfluss unterschiedlicher Mengen lernen.

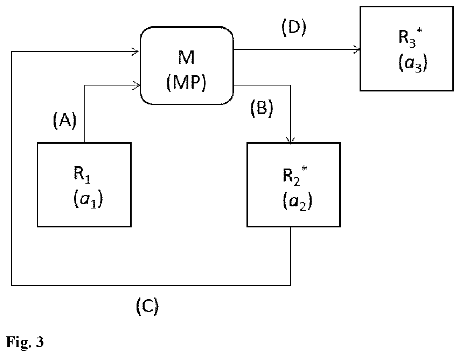

Ist das in Fig. 2 gezeigte Modell des maschinellen Lernens trainiert, kann es zur Vorhersage verwendet werden. Dies ist in Fig. 3 dargestellt. In einem ersten Schritt (A) wird eine erste Repräsentation $R_1$ dem trainierten Modell M des maschinellen Lernens zugeführt. Die erste Repräsentation $R_1$ repräsentiert den Untersuchungsbereich eines neuen Untersuchungsobjekts nach der Applikation der ersten Menge $a_1$ eines Kontrastmittels (wobei die erste Menge $a_1$ auch Null sein kann). Dabei kann der Begriff "neu" bedeuten, dass von dem Untersuchungsobjekt keine Repräsentationen beim Trainieren des Modells M des maschinellen Lernens verwendet wurden. Das Modell M erzeugt in Schritt (B) auf Basis der ersten Repräsentation $R_1$ und auf Basis der Modellparameter MP eine zweite Repräsentation $R_2^*$. Die zweite Repräsentation $R_2^*$ repräsentiert den Untersuchungsbereichs des Untersuchungsobjekts nach der Applikation der zweiten Menge $a_2$. In einem dritten Schritt (C) wird die erzeugte zweite Repräsentation $R_2^*$ dem Modell M des maschinellen Lernens zugeführt. Das Modell M erzeugt in Schritt (D) auf Basis der zweiten Repräsentation $R_2^*$ und auf Basis der Modellparameter MP eine dritte Repräsentation $R_3^*$. Die dritte Repräsentation $R_3^*$ repräsentiert den Untersuchungsbereichs des Untersuchungsobjekts nach der Applikation der dritten Menge $a_3$ an Kontrastmittel.

Das in Fig. 3 dargestellte Vorhersageverfahren, das auf dem in Fig. 2 dargestellten Trainingsverfahren beruht, ist eine bevorzugte Ausführungsform der vorliegenden Offenbarung und umfasst die Schritte:

- Empfangen einer Repräsentation $R_1$ eines Untersuchungsbereichs, wobei die Repräsentation $R_1$ den Untersuchungsbereich ohne Kontrastmittel oder nach Applikation einer ersten Menge $a_1$ eines Kontrastmittels repräsentiert,

- Erzeugen einer Repräsentation $R_2^*$ des Untersuchungsbereichs mit Hilfe eines trainierten Modells des maschinellen Lernens zumindest anteilig auf Basis der Repräsentation $R_1$, wobei die erzeugte Repräsentation $R_2^*$ den Untersuchungsbereich nach Applikation einer zweiten Menge $a_2$ des Kontrastmittels repräsentiert, wobei die zweite Menge $a_2$ vorzugsweise größer als Null und vorzugsweise größer als die erste Menge $a_1$ ist,

- Erzeugen einer Repräsentation $R_3^*$ des Untersuchungsbereichs mit Hilfe des Modells des maschinellen Lernens zumindest anteilig auf Basis der erzeugten Repräsentation $R_2^*$, wobei die erzeugte Repräsentation $R_3^*$ den Untersuchungsbereich nach Applikation einer dritten Menge $a_3$ des Kontrastmittels repräsentiert, wobei die dritte Menge $a_3$ vorzugsweise größer als die zweite Menge $a_2$ ist,

- Ausgeben und/oder Speichern und/oder Übermitteln der Repräsentation $R_3^*$,

wobei das Modell des maschinellen Lernens auf Basis von Trainingsdaten trainiert wurde, wobei die Trainingsdaten eine Vielzahl von Datensätzen umfasst, wobei jeder Datensatz eine erste Repräsentation $^{T}R_1$, eine zweite Repräsentation $^{T}R_2$ und eine dritte Repräsentation $^{T}R_3$ umfasst, wobei die erste Repräsentation $^{T}R_1$ den Untersuchungsbereich ohne Kontrastmittel oder nach der Applikation der ersten Menge $a_1$ des Kontrastmittels repräsentiert, die zweite Repräsentation $^{T}R_2$ den Untersuchungsbereich nach der Applikation der zweiten Menge $a_2$ des Kontrastmittels repräsentiert und die dritte Repräsentation $^{T}R_3$ den Untersuchungsbereich nach der Applikation der dritten Menge $a_3$ des Kontrastmittels repräsentiert, wobei das Modell des maschinellen Lernens trainiert wurde, zumindest anteilig auf Basis der ersten Repräsentation $^{T}R_1$ eine Repräsentation $^{T}R_2^*$ zu erzeugen und zumindest anteilig auf Basis der erzeugten Repräsentation $^{T}R_2^*$ eine Repräsentation $^{T}R_3^*$ zu erzeugen, wobei beim Trainieren Abweichungen zwischen den Repräsentationen $^{T}R_2$ und $^{T}R_2^*$ sowie zwischen den Repräsentationen $^{T}R_3$ und $^{T}R_3^*$ quantifiziert werden und Modellparameter modifiziert werden, um die Abweichungen zu minimieren.

Allgemein kann das Modell des maschinellen Lernens trainiert werden, ausgehend von einer ersten Repräsentation $^{T}R_1$ nacheinander eine Zahl $n-1$ von Repräsentationen $^{T}R_2^*$ bis $^{T}R_n^*$ zu erzeugen, wobei $n$ eine ganze Zahl größer als 2 ist, wobei die erste Repräsentation $^{T}R_1$ den Untersuchungsbereich ohne Kontrastmittel oder nach Applikation einer ersten Menge $a_1$ eines Kontrastmittels repräsentiert und jede erzeugte Repräsentation $^{T}R_j^*$ den Untersuchungsbereich nach einer Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^{T}R_2^*$ zumindest anteilig auf Basis der Repräsentation $^{T}R_1$ erfolgt und die Erzeugung jeder weiteren Repräsentation $^{T}R_k^*$ zumindest anteilig auf Basis der jeweils zuvor erzeugten Repräsentation $^{T}R_{k-1}^*$ erfolgt, wobei $k$ ein Index ist, der ganze Zahlen von 3 bis $n$ durchläuft.

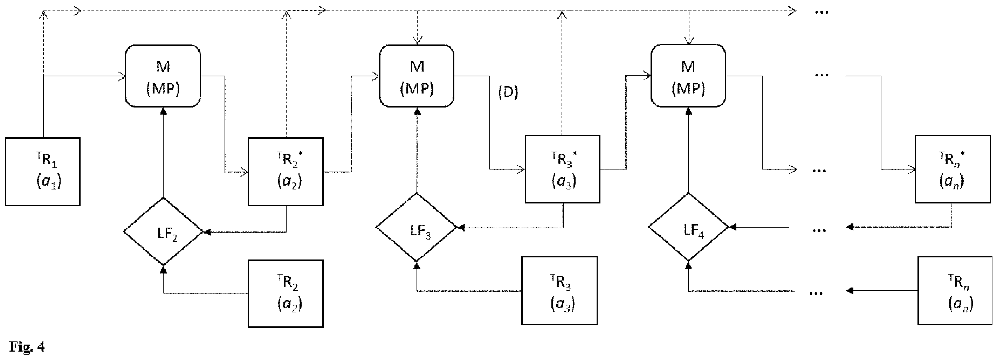

Dies sei am Beispiel von Fig. 4 näher erläutert. Fig. 4 kann als eine Erweiterung des in Fig. 2 gezeigten Schemas von drei Repräsentationen auf eine (zumindest innerhalb definierter Grenzen) beliebige Zahl $n$ an Repräsentationen verstanden werden, wobei $n$ eine ganze Zahl größer als 2 ist. Vorzugsweise liegt die Zahl $n$ im Bereich von 3 bis 100. Die Zahl $n$ kann jedoch auch größer als 100 sein.

Fig. 4 zeigt ein Modell M des maschinellen Lernens. Das Modell M des maschinellen Lernens ist dreimal aufgeführt; es handelt sich aber stets um dasselbe Modell. Das Modell M des maschinellen Lernens wird trainiert, nacheinander eine Zahl $n-1$ an Repräsentationen vorherzusagen. Im vorliegenden Beispiel ist $n$ eine ganze Zahl größer als 3. Dem Modell M wird eine erste Repräsentation $^{T}R_1$ als Eingabedaten zugeführt. Die erste Repräsentation $^{T}R_1$ repräsentiert einen Untersuchungsbereich eines Untersuchungsobjekts ohne Kontrastmittel oder nach der Applikation einer ersten Menge $a_1$ eines Kontrastmittels. Das Modell M des maschinellen Lernens ist konfiguriert, zumindest anteilig auf Basis der ersten Repräsentation $^{T}R_1$ und auf Basis von Modellparametern MP eine Ausgabe $^{T}R_2^*$ zu erzeugen. Die Ausgabe $^{T}R_2^*$ stellt eine vorhergesagte zweite Repräsentation dar, die den Untersuchungsbereich nach Applikation einer zweiten Menge $a_2$ des Kontrastmittels repräsentiert. Das Modell ist ferner konfiguriert, zumindest anteilig auf Basis der Ausgabe $^{T}R_2^*$ und Modellparametern MP eine Ausgabe $^{T}R_3^*$ zu erzeugen. Die Ausgabe $^{T}R_3^*$ stellt eine vorhergesagte dritte Repräsentation dar, die den Untersuchungsbereich nach Applikation einer dritten Menge $a_3$ des Kontrastmittels repräsentiert. Das Modell ist ferner konfiguriert, zumindest anteilig auf Basis der Ausgabe $^{T}R_3^*$ und Modellparametern MP eine weitere Ausgabe zu erzeugen, die eine vorhergesagte Repräsentation des Untersuchungsbereichs nach einer Applikation einer weiteren Menge des Kontrastmittels repräsentiert. Dieses Schema wird bis zu einer Ausgabe $^{T}R_n^*$ fortgesetzt. Die Ausgabe $^{T}R_n^*$ stellt eine vorhergesagte n-te Repräsentation dar, die den Untersuchungsbereich nach der Applikation einer n-ten Menge $a_n$ des Kontrastmittels repräsentiert. Die Mengen an Kontrastmittel $a_1$, $a_2$, $a_3$, … , $a_n$ bilden eine Folge. Bei der Folge kann es sich beispielsweise um eine Folge zunehmender Kontrastmittelmengen handeln. Mit anderen Worten: vorzugsweise ist die zweite Menge $a_2$ größer als Null und größer als die erste Menge $a_1$, die dritte Menge $a_3$ größer als die zweite Menge $a_2$, die vierte Menge $a_4$ größer als die dritte Menge $a_3$ und so fort. Allgemein ausgedrückt: die j-te Menge $a_j$ ist vorzugsweise größer als die (j-1)-te Menge $a_{j-1}$, wobei $j$ ein Index ist, der die Zahlen von 2 bis $n$ durchläuft.

Im Fall des in Fig. 4 gezeigten Beispiels liegen, neben der ersten Repräsentation $^{T}R_1$ weitere (reale, messtechnisch erzeugte) Repräsentationen $^{T}R_j$ (mit $j$ = 2 bis $n$) des Untersuchungsbereichs nach der Applikation der Mengen $a_j$ (mit $j$ = 2 bis $n$) des Kontrastmittels vor, die als Zieldaten (*ground truth*) zum Trainieren des Modells des maschinellen Lernens verwendet werden können. So kann die Abweichung zwischen einer Ausgabe $^{T}R_j^*$ (die eine vorgesagte j-te Repräsentation des Untersuchungsbereichs nach der Applikation einer Menge $a_j$ des Kontrastmittels darstellt) und der Repräsentation $^{T}R_j$ mit einer Fehlerfunktion $LF_j$ quantifiziert werden: eine Fehlerfunktion $LF_2$ kann die Abweichung zwischen der Repräsentation $^{T}R_2$ und der erzeugten Repräsentation $^{T}R_2^*$ quantifizieren, eine Fehlerfunktion $LF_3$ kann die Abweichung

zwischen der Repräsentation $^T R_3$ und der erzeugten Repräsentation $^T R_3^*$ quantifizieren, und so fort.

In einem Ende-zu-Ende-Training kann eine Gesamtfehlerfunktion LF verwendet werden, die alle einzelnen Abweichungen berücksichtigt; dabei kann es sich beispielsweise um die Summe der einzelnen Abweichungen handeln:

$$LF = LF_2 + LF_3 + \ldots + LF_n = \sum_{j=2}^{n} LF_j$$

Es kann sich aber auch um eine gewichtete Summe handeln:

$$LF = w_2 \cdot LF_2 + w_3 \cdot LF_3 + \ldots + w_n \cdot LF_n = \sum_{j=2}^{n} w_j \cdot LF_j$$

wobei die Gewichtsfaktoren $w_j$ beispielsweise Werte von 0 bis 1 annehmen können.

Die Gewichtung hat den Vorteil, dass den Repräsentationen, die dem Modell des maschinellen Lernens zugeführt werden, unterschiedliches Gewicht bei der Erzeugung einer vorhergesagten Repräsentation beigemessen wird.

So ist es beispielweise möglich, dass die Gewichtsfaktoren $w_j$ in der Reihe $w_2$, $w_3$, ..., $w_n$ mit der Menge an Kontrastmittel $a_2$, $a_3$, ..., $a_n$ zunehmen und damit Repräsentationen, deren Menge an Kontrastmittel näher an der Menge $a_n$ liegt, höher gewichtet werden. Dabei kann die Zunahme logarithmisch, linear, quadratisch, kubisch oder exponentiell sein oder eine andere Zunahme sein. Es ist auch denkbar, den Repräsentationen mit einer größeren Menge an Kontrastmittel weniger Gewicht beizumessen, indem die Gewichtsfaktoren in der Reihe $w_2$, $w_3$, ..., $w_n$ mit zunehmender Menge an Kontrastmittel $a_2$, $a_3$, ..., $a_n$ abnehmen und damit Repräsentationen, die näher an der initialen Repräsentation $R_1$ liegen, mehr Gewicht beimessen. Auch eine solche Abnahme kann logarithmisch, linear, quadratisch, kubisch oder exponentiell sein oder eine andere Abnahme sein.

Es ist auch denkbar, dass die Trainingsdaten unvollständige Datensätze umfassen. Das bedeutet, dass einige Datensätze von Untersuchungsobjekten nicht alle Repräsentationen $^T R_1$ bis $^T R_n$ umfassen. Wie weiter hinten in der Beschreibung erläutert, können auch unvollständige Datensätze zum Trainieren des Modells des maschinellen Lernens verwendet werden. Falls eine Repräsentation $^T R_j$ fehlt, kann der Gewichtsfaktor $w_j$, der die Fehlerfunktion zu der Repräsentation $^T R_j$ und der erzeugten Repräsentation $^T R_j^*$ gewichten soll, auf Null gesetzt werden, wobei $j$ eine ganze Zahl ist, die die Werte 2 bis $n$ annehmen kann.

Es ist auch denkbar, dass bei dem Training zufällige Zahlen $1 \leq j < k \leq n$ und die damit verbundenen Teilfolgen von Kontrastmittelmengen $a_j$, $a_{j+1}$, ..., $a_{k-1}$, $a_k$ betrachtet werden. Das oben beschriebene Lernproblem kann dann auf diesen (optional von Zeit zu Zeit variierenden) Teilfolgen gelöst werden. Zum Beispiel ist denkbar, dass eine zufällige initiale Kontrastmittelmenge $a_j$ ($1 \leq j \leq n$-2) bestimmt wird und das Modell auf dessen Basis stets die Repräsentationen der nachfolgenden zwei Kontrastmittelmengen $a_{j+1}$, $a_{j+2}$ synthetisieren soll.

In Fig. 4 ist auch schematisch dargestellt, dass zur Erzeugung einer Repräsentation $^T R_j^*$, die den Untersuchungsbereich nach der Applikation einer Menge $a_j$ des Kontrastmittels repräsentiert, neben der Repräsentation $^T R_{j-1}^*$, die den Untersuchungsbereich nach der Applikation einer Menge $a_{j-1}$ repräsentiert, auch weitere Repräsentation $^T R_{j-2}^*$ und/oder $^T R_{j-3}^*$ und/oder ... bis $^T R_1$, die den Untersuchungsbereich nach der Applikation der weiteren Mengen $a_{j-2}$, $a_{j-3}$ ... bis $a_1$ repräsentieren, verwendet werden können, wobei $j$ eine ganze Zahl ist, die die Werte 2 bis $n$ annehmen kann. Dies wird durch die gestrichelten Pfeile zum Ausdruck gebracht. So kann zur Erzeugung der Repräsentation $^T R_3^*$ neben der Repräsentation $^T R_2^*$ auch die Repräsentation $^T R_1$ dem Modell des maschinellen Lernens zugeführt werden. Zur Erzeugung der Repräsentation $^T R_4^*$ kann neben der Repräsentation $^T R_3^*$ auch die Repräsentation $^T R_2^*$ und/oder die Repräsentation $^T R_1$ dem Modell des maschinellen Lernens zugeführt werden.

Es können zur Vorhersage von Repräsentationen auch weitere Informationen in das Modell des maschinellen Lernens einfließen, wie beispielsweise Informationen über die Menge an Kontrastmittel, die jeweils appliziert worden ist. Mit anderen Worten: neben der ersten Repräsentation können zur Vorhersage der zweiten Repräsentation auch Informationen zu der Menge an appliziertem Kontrastmittel, die durch die erste Repräsentation repräsentiert wird, verwendet werden. Auch die vorhergesagte dritte Repräsentation kann auf Basis der vorhergesagten zweiten Repräsentation und Informationen zu der zweiten Menge an Kontrastmittel erzeugt werden. Wird eine Informationen über die Menge an Kontrastmittel einer Repräsentation mitgegeben, so "weiß" das Modell des maschinellen Lernens, an welcher Stelle der Folge von Kontrastmittelmengen es sich jeweils befindet.

Weitere Informationen, die zum Trainieren und zur späteren Vorhersage des Modells des maschinellen Lernens verwendet werden können, sind z.B. Informationen zum Untersuchungsbereich, zum Untersuchungsobjekt, zu Bedingungen, die bei der Erzeugung der Repräsentation herrschten, Informationen zu dem Kontrastmittel, Informationen zu der Kontrastmittelmenge, die dem Untersuchungsobjekt appliziert worden ist, und andere/weitere.

Das Modell des maschinellen Lernens der vorliegenden Offenbarung kann als eine Transformation verstanden werden, die auf eine Repräsentation eines Untersuchungsbereichs in einem Zustand einer Folge von Zuständen angewendet werden kann, um eine Repräsentation des Untersuchungsbereichs in einem nachfolgenden Zustand der Folge von Zuständen vorherzusagen. Jeder Zustand wird durch eine Menge an appliziertem Kontrastmittel repräsentiert. Die Transformation kann einfach (einmal) angewendet werden, um eine Repräsentation des Untersuchungsbereichs in dem unmittelbar nachfolgenden Zustand vorherzusagen, oder mehrfach (mehrmals) angewendet werden, um eine Repräsentation des Untersuchungsbereichs in einem in der Folge der Zustände weiter hinten liegenden Zustand vorherzusagen.

Liegt eine Folge von $n$ Zuständen $Z_1$ bis $Z_n$ vor, und liegt zu jedem Zustand $Z_i$ eine Repräsentation $R_i$ vor, die den Untersuchungsbereich in dem Zustand $Z_i$ repräsentiert, so kann das Modell M des maschinellen Lernens ausgehend von der Repräsentation $R_1$ q-fach angewendet werden, um eine Repräsentation $R_{1+q}$ zu erzeugen, die den Untersuchungsbereich in dem Zustand $Z_{1+q}$ repräsentiert, wobei q die Werte 1 bis $n$-1 annehmen kann:

$$\mathbf{M}^q\,(\mathrm{R}_1) = \mathrm{R}_{1+q}{}^*$$

$$q = 1: \mathbf{M}(\mathrm{R}_1) = \mathrm{R}_2{}^*$$

$$q = 2: \mathbf{M}(\mathrm{R}_2{}^*) = \mathbf{M}(\mathbf{M}(\mathrm{R}_1)) = \mathbf{M}^2(\mathrm{R}_1) = \mathrm{R}_3{}^*$$

$$q = 3: \mathbf{M}(\mathrm{R}_3{}^*) = \mathbf{M}(\mathbf{M}(\mathrm{R}_2{}^*)) = \mathbf{M}(\mathbf{M}(\mathbf{M}(\mathrm{R}_1))) = \mathbf{M}^3(\mathrm{R}_1) = \mathrm{R}_4{}^*$$

$$\dots$$

$$q = n\text{-}1: \mathbf{M}(\mathrm{R}_{n-1}{}^*) = \mathbf{M}(\mathbf{M}(\mathrm{R}_{n-2}{}^*)) = \dots = \mathbf{M}^{n-1}(\mathrm{R}_1) = \mathrm{R}_n{}^*$$

Dies gilt analog auch für das Trainingsverfahren, bei dem die q-fache Transformation M durch die folgende Formel beschrieben werden kann:

$$\mathbf{M}^q\,({}^{\mathrm{T}}\mathrm{R}_1) = {}^{\mathrm{T}}\mathrm{R}_{1+q}{}^*$$

Der jeweilige Zustand gibt an, welche Kontrastmittelmenge appliziert worden ist. Eine Folge von Zuständen kann also beispielsweise den Untersuchungsbereich nach Applikation einer Folge von zunehmenden Kontrastmittelmengen charakterisieren.

Eine Fehlerfunktion, die alle Abweichungen zwischen vorhergesagten Repräsentationen ${}^{\mathrm{T}}\mathrm{R}_q{}^*$ und realen (messtechnisch erzeugten) Repräsentationen ${}^{\mathrm{T}}\mathrm{R}_q$ quantifiziert, kann beispielsweise durch die folgende Formel ausgedrückt werden:

$$\mathrm{LV} = \mathrm{w}_2 \cdot \mathbf{d}(\mathbf{M}({}^{\mathrm{T}}\mathrm{R}_1),\ {}^{\mathrm{T}}\mathrm{R}_2) + \mathrm{w}_3 \cdot \mathbf{d}(\mathbf{M}^2({}^{\mathrm{T}}\mathrm{R}_1),\ {}^{\mathrm{T}}\mathrm{R}_3) + \dots + \mathrm{w}_n \cdot \mathbf{d}(\mathbf{M}^{n-1}({}^{\mathrm{T}}\mathrm{R}_1),\ {}^{\mathrm{T}}\mathrm{R}_n)$$

Dabei ist LV der Fehlerwert, der sich für einen Datensatz umfassend die (realen, messtechnisch erzeugten) Repräsentationen ${}^{\mathrm{T}}\mathrm{R}_1, {}^{\mathrm{T}}\mathrm{R}_2, ..., {}^{\mathrm{T}}\mathrm{R}_n$ ergibt. $\mathbf{d}$ ist eine Fehlerfunktion, die die Abweichungen zwischen einer vorhergesagten Repräsentation $\mathbf{M}({}^{\mathrm{T}}\mathrm{R}_{q-1})$ und der Repräsentation ${}^{\mathrm{T}}\mathrm{R}_q$ quantifiziert. Wie bereits weiter vorne in der Beschreibung beschrieben, kann es sich dabei beispielsweise um eine der folgenden Fehlerfunktionen handeln: L1 loss, L2 loss, Lp loss, Strukturähnlichkeitsindexmaß (SSIM), *VGG loss, perceptual loss* oder eine Kombination davon.

$\mathrm{w}_2, \mathrm{w}_3, ..., \mathrm{w}_n$ sind Gewichtsfaktoren, die ebenfalls weiter vorne in der Beschreibung bereits beschrieben wurden.

$n$ gibt die Zahl der Zustände (die Zahl der unterschiedlichen Mengen an Kontrastmittel) an.

Es ist auch denkbar, dass der Fehlerwert die maximale Abweichung ist, die für einen Datensatz berechnet wird:

$$\mathrm{LV} = \max(\mathrm{w}_2 \cdot \mathbf{d}(\mathbf{M}({}^{\mathrm{T}}\mathrm{R}_1),\ {}^{\mathrm{T}}\mathrm{R}_2);\ \mathrm{w}_3 \cdot \mathbf{d}(\mathbf{M}^2({}^{\mathrm{T}}\mathrm{R}_1),\ {}^{\mathrm{T}}\mathrm{R}_3);\ \dots;\ \mathrm{w}_n \cdot \mathbf{d}(\mathbf{M}^{n-1}({}^{\mathrm{T}}\mathrm{R}_1),\ {}^{\mathrm{T}}\mathrm{R}_n))$$

wobei auch in dieser Formel durch die Gewichtsfaktoren den einzelnen Abweichungen ein unterschiedliches Gewicht beigemessen werden kann.

Wie bereits weiter vorne in der Beschreibung angedeutet, sei angemerkt, dass es zum Trainieren des Modells des maschinellen Lernens nicht erforderlich ist, dass jeder Datensatz der Trainingsdaten Repräsentationen von allen Kontrastmittelmengen, die das Modell lernen soll, beinhaltet. Prinzipiell sind zwei Repräsentationen des Untersuchungsbereichs nach der Applikation von zwei unterschiedlichen Mengen an Kontrastmittel ausreichend. Dies sei an einem Beispiel erläutert. Es sei angenommen, dass das Modell des maschinellen Lernens trainiert werden soll, Repräsentationen eines Untersuchungsbereichs mit den zunehmenden Kontrastmittelmengen $a_1$ bis $a_6$ vorherzusagen. Es sei angenommen, dass zum Trainieren des Modells des maschinellen Lernens Trainingsdaten ausreichend sind, die 10 Datensätze von 10 Untersuchungsobjekten umfassen. Jeder Datensatz umfasst Repräsentationen des Untersuchungsbereichs nach Applikation verschiedener Mengen an Kontrastmittel, z.B.:

Datensatz 1: $^{T}R_1$, $^{T}R_3$, $^{T}R_4$, $^{T}R_5$, $^{T}R_6$
Datensatz 2: $^{T}R_1$, $^{T}R_2$, $^{T}R_4$, $^{T}R_6$
Datensatz 3: $^{T}R_1$, $^{T}R_2$, $^{T}R_3$, $^{T}R_4$, $^{T}R_5$
Datensatz 4: $^{T}R_1$, $^{T}R_2$, $^{T}R_3$, $^{T}R_5$, $^{T}R_6$
Datensatz 5: $^{T}R_2$, $^{T}R_3$, $^{T}R_5$, $^{T}R_6$
Datensatz 6: $^{T}R_2$, $^{T}R_3$, $^{T}R_4$, $^{T}R_5$, $^{T}R_6$
Datensatz 7: $^{T}R_2$, $^{T}R_3$, $^{T}R_5$, $^{T}R_6$
Datensatz 8: $^{T}R_3$, $^{T}R_5$, $^{T}R_6$
Datensatz 9: $^{T}R_3$, $^{T}R_4$, $^{T}R_5$, $^{T}R_6$
Datensatz 10: $^{T}R_3$, $^{T}R_4$, $^{T}R_6$

In dem Beispiel gibt es keinen einzigen "vollständigen" Datensatz, also keinen Datensatz, der alle möglichen Repräsentationen $^{T}R_1$, $^{T}R_2$, $^{T}R_3$, $^{T}R_4$, $^{T}R_5$ und $^{T}R_6$ umfasst. Dennoch ist es möglich, das Modell des maschinellen Lernens auf Basis von solchen Trainingsdaten zu trainieren, eine Repräsentation des Untersuchungsbereichs nach Applikation einer jeden Menge $a_2$ bis $a_6$ vorherzusagen. Dies ist ein weiterer Vorteil der vorliegenden Erfindung gegenüber dem oben beschriebenen "direkten Trainieren".

Ist das Modell des maschinellen Lernens trainiert, können dem Modell neue (d.h. nicht im Training verwendete) Repräsentationen eines Untersuchungsbereichs nach Applikation einer Menge des Kontrastmittels zugeführt werden, und das Modell kann eine oder mehrere Repräsentationen des Untersuchungsbereichs nach Applikation einer anderen (größeren) Menge vorhersagen (erzeugen).

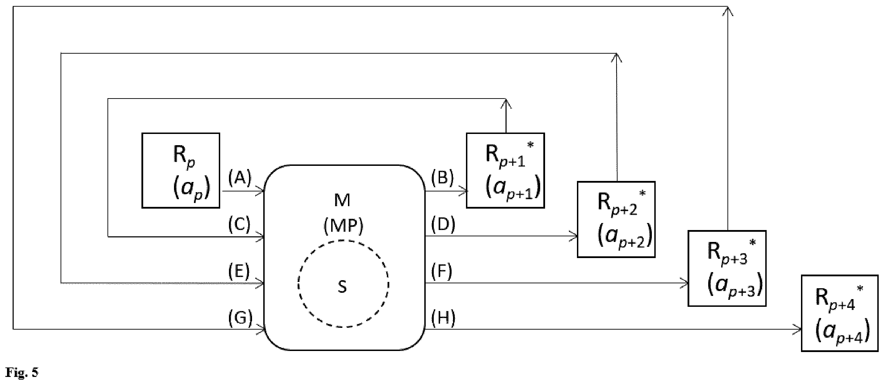

Dies ist beispielhaft und schematisch in Fig. 5 dargestellt. In dem in Fig. 5 dargestellten Beispiel werden ausgehend von einer Repräsentation $R_p$, die den Untersuchungsbereich nach der Applikation der Menge $a_p$ eines Kontrastmittels repräsentiert, eine Folge von Repräsentationen $R_{p+1}^*$, $R_{p+2}^*$, $R_{p+3}^*$ und $R_{p+4}^*$ erzeugt, die den Untersuchungsbereich nach der Applikation der Mengen $a_{p+1}$, $a_{p+2}$, $a_{p+3}$ und $a_{p+4}$ repräsentieren. Die Kontrastmittelmengen $a_{p+1}$, $a_{p+2}$, $a_{p+3}$ und $a_{p+4}$ bilden vorzugsweise eine Folge von zunehmenden Mengen: $a_{p+1} < a_{p+2} < a_{p+3} < a_{p+4}$.

In Schritt (A) wird dem Modell des maschinellen Lernens M die Repräsentation $R_p$ zugeführt. Neben der Repräsentation $R_p$ können dem Modell des maschinellen Lernens M auch Informationen zu der Kontrastmittelmenge $a_p$ und/oder weitere/andere Informationen zugeführt werden, wie weiter vorne in dieser Beschreibung beschrieben.

Das Modell des maschinellen Lernens M erzeugt in Schritt (B) auf Basis der zugeführten Eingabedaten die Repräsentation $R_{p+1}^*$, die den Untersuchungsbereich nach der Applikation der Menge $a_{p+1}$ repräsentiert.

In Schritt (C) wird dem Modell des maschinellen Lernens M die zuvor erzeugte Repräsentation $R_{p+1}^*$ zugeführt. Neben der Repräsentation $R_{p+1}^*$ können dem Modell des maschinellen Lernens M auch Informationen zu der Kontrastmittelmenge $a_{p+1}$ und/oder weitere Informationen zugeführt werden. Ferner können dem Modell des maschinellen Lernens auch die Repräsentation $R_p$ und/oder Informationen zu der Kontrastmittelmenge $a_p$ zugeführt werden.

Vorzugsweise umfasst das Modell des maschinellen Lernens M einen Speicher S, der Eingabedaten (und vorzugsweise auch Ausgabedaten) speichert, so dass einmal eingegebene Eingabedaten und/oder erzeugte Ausgabedaten nicht erneut empfangen und/oder eingegeben werden müssen, sondern dem Modell des maschinellen Lernens bereits zur Verfügung stehen. Dies gilt nicht nur für die in diesem Beispiel beschriebene Nutzung des trainierten Modells des maschinellen Lernens zur Vorhersage, sondern auch für das Trainieren des erfindungsgemäßen Modells des maschinellen Lernens und allgemein auch für alle andere Ausführungsformen.

Das Modell des maschinellen Lernens M erzeugt in Schritt (D) auf Basis der zugeführten Eingabedaten die Repräsentation $R_{p+2}^*$, die den Untersuchungsbereich nach der Applikation der Kontrastmittelmenge $a_{p+2}$ repräsentiert.

In Schritt (E) wird dem Modell des maschinellen Lernens M die zuvor erzeugte Repräsentation $R_{p+2}^*$ zugeführt. Neben der Repräsentation $R_{p+2}^*$ können dem Modell des maschinellen Lernens M auch Informationen zu der Kontrastmittelmenge $a_{p+2}$ und/oder weitere Informationen zugeführt werden. Ferner können dem Modell des maschinellen Lernens auch die Repräsentation $R_p$ und/oder die Repräsentation $R_{p+1}^*$ und/oder Informationen zu den Kontrastmittelmengen $a_p$ und/oder $a_{p+1}$ zugeführt werden.

Das Modell des maschinellen Lernens M erzeugt in Schritt (F) auf Basis der zugeführten Eingabedaten die Repräsentation $R_{p+3}^*$, die den Untersuchungsbereich nach der Applikation der Kontrastmittelmenge $a_{p+3}$ repräsentiert.

In Schritt (G) wird dem Modell des maschinellen Lernens M die zuvor erzeugte Repräsentation $R_{p+3}^*$ zugeführt. Neben der Repräsentation $R_{p+3}^*$ können dem Modell des maschinellen Lernens M auch Informationen zu der Kontrastmittelmenge $a_{p+3}$ und/oder weitere Informationen zugeführt werden. Ferner können dem Modell des maschinellen Lernens auch die Repräsentation $R_p$ und/oder die Repräsentation $R_{p+1}^*$ und/oder die Repräsentation $R_{p+2}^*$ und/oder Informationen zu den Kontrastmittelmengen $a_p$ und/oder $a_{p+1}$ und/oder $a_{p-2}$ zugeführt werden.

Das Modell des maschinellen Lernens M erzeugt in Schritt (H) auf Basis der zugeführten Eingabedaten die Repräsentation $R_{p+4}^*$, die den Untersuchungsbereich nach der Applikation der Kontrastmittelmenge $a_{p+4}$ repräsentiert.

Die erzeugten Repräsentationen $R_{p+1}^*$, $R_{p+2}^*$, $R_{p+3}^*$ und/oder $R_{p+4}^*$ können ausgegeben (z.B. auf einem Monitor angezeigt und/oder mit einem Drucker ausgedruckt) und/oder in einem Datenspeicher gespeichert und/oder an ein (separates) Computersystem übermittelt werden.

Wurde das erfindungsgemäße Modell des maschinellen Lernens trainiert, ausgehend von einer ersten Repräsentation $^TR_1$, die den Untersuchungsbereich nach der Applikation einer ersten Menge $a_1$ des Kontrastmittels repräsentiert, eine Folge von vorhergesagten Repräsentationen $^TR_2^*$, $^TR_3^*$, ..., $^TR_n^*$ zu erzeugen, wobei jede vorhergesagte Repräsentation $^TR_j^*$ den Untersuchungsbereich nach der Applikation einer Menge $a_j$ des Kontrastmittels repräsentiert, wobei $j$ ein Index ist, der ganze Zahlen von 2 bis $n$ durchläuft, dann kann einem solchen Modell eine neue Repräsentation $R_p$ zugeführt werden, die den Untersuchungsbereich nach der Applikation der Menge $a_p$ des Kontrastmittels repräsentiert, und das Modell des maschinellen Lernens kann nacheinander die vorhergesagten Repräsentationen $R_{p+1}^*$, $R_{p+2}^*$, ..., $R_n^*$ erzeugen, wobei $p$ eine Zahl ist, die die Werte 2 bis $n$ annehmen kann.

Das bedeutet, dass dem trainierten Modell nicht zwangsläufig eine neue Repräsentation $R_1$, die den Untersuchungsbereich nach der Applikation der ersten Menge $a_1$ repräsentiert, zugeführt werden muss, und das trainierte Modell des maschinellen Lernens ist auch nicht darauf beschränkt, nur die Repräsentation $R_n$, die den Untersuchungsbereich nach der Applikation der (vorzugsweise größten) Menge $a_n$ repräsentiert, vorherzusagen. Stattdessen kann man in der Folge von Kontrastmittelmengen $a_1$ bis $a_n$ an jeder Stelle "einsteigen" und "aussteigen", d.h. auf Basis einer beliebigen Repräsentation zu der Folge von Kontrastmittelmengen eine beliebige andere Repräsentation zu einer in der Folge der Konzentrationsmengen nachfolgenden Konzentrationsmenge vorhersagen.

Ist also ein Modell des maschinellen Lernens beispielsweise trainiert worden, den Effekt einer zunehmenden Menge an Kontrastmittel in einer Folge zunehmender Kontrastmittelmengen $a_1$, $a_2$, $a_3$, $a_4$ zu lernen, dann kann dem trainierten Modell des maschinellen Lernens beispielsweise

- eine Repräsentation $R_1$ zugeführt werden, um eine Repräsentation $R_2^*$ und/oder $R_3^*$ und/oder $R_4^*$ zu erzeugen, oder
- eine Repräsentation $R_2$ zugeführt werden, um eine Repräsentation $R_3^*$ und/oder $R_4^*$ zu erzeugen, oder
- eine Repräsentation $R_3$ zugeführt werden, um eine Repräsentation $R_4^*$ zu erzeugen,

wobei die Repräsentation $R_1$ den Untersuchungsbereich nach der Applikation der Menge $a_1$ repräsentiert, die Repräsentationen $R_2$ und $R_2^*$ den Untersuchungsbereich nach der Applikation der Menge $a_2$ repräsentiert, die Repräsentationen $R_3$ und $R_3^*$ den Untersuchungsbereich nach der Applikation der Menge $a_3$ repräsentiert und die Repräsentationen $R_4$ und $R_4^*$ den Untersuchungsbereich nach der Applikation der Menge $a_4$ repräsentiert.

Es ist sogar möglich, Repräsentationen zu erzeugen, die den Untersuchungsbereich nach Applikation einer Kontrastmittelmenge repräsentieren, die gar nicht Gegenstand des Trainings gewesen ist. Anstatt also beim der Repräsentation $R_n^*$ (z.B. im obigen Beispiel $R_4^*$) zu stoppen, können auch eine Repräsentation $R_{n+1}^*$ (z.B. $R_5^*$), $R_{n+2}^*$ (z.B. $R_6^*$) und so weiter erzeugt werden. Das trainierte Modell des maschinellen Lernens kann also verwendet werden, um den gelernten Effekt einer vorzugsweise zunehmenden Kontrastmittelmenge fortzusetzen und Repräsentationen zu berechnen, die messtechnisch niemals erzeugt worden sind. Insofern kann das trainierte Modell des maschinellen Lernens zur Extrapolation auf sehr hohe Kontrastmittelmengen verwendet werden, die man einem Untersuchungsobjekt niemals applizieren würde.

Ferner sind die Vorhersagen nicht auf zunehmende Kontrastmittelmengen beschränkt. Es ist auch möglich, Repräsentationen mit abnehmenden Kontrastmittelmengen vorherzusagen. Zum einen kann das Modell des maschinellen Lernens grundsätzlich in beide Richtungen trainiert werden: in Richtung zunehmender Kontrastmittelmengen und in Richtung abnehmender Kontrastmittelmengen. Zum anderen führt das Modell des maschinellen Lernens an einer in das Modell eingegebenen Repräsentation eine Transformation aus, die sich prinzipiell auch umkehren lässt. Aus der Analyse der gelernten mathematischen Funktionen des Modells, die eine Eingabe-Repräsentation in eine Ausgabe-Repräsentation transformieren, lassen sich Umkehrfunktionen ermitteln, die den Prozess umkehren und die vorherige Ausgabe-Repräsentation wieder in die vorherige Eingabe-Repräsentation zurückverwandelt. Die Umkehrfunktionen können dann verwendet werden, Repräsentationen mit abnehmenden Kontrastmittelmengen vorherzusagen, auch wenn das Modell trainiert worden ist, Repräsentationen mit zunehmenden Kontrastmittelmengen vorherzusagen und umgekehrt.

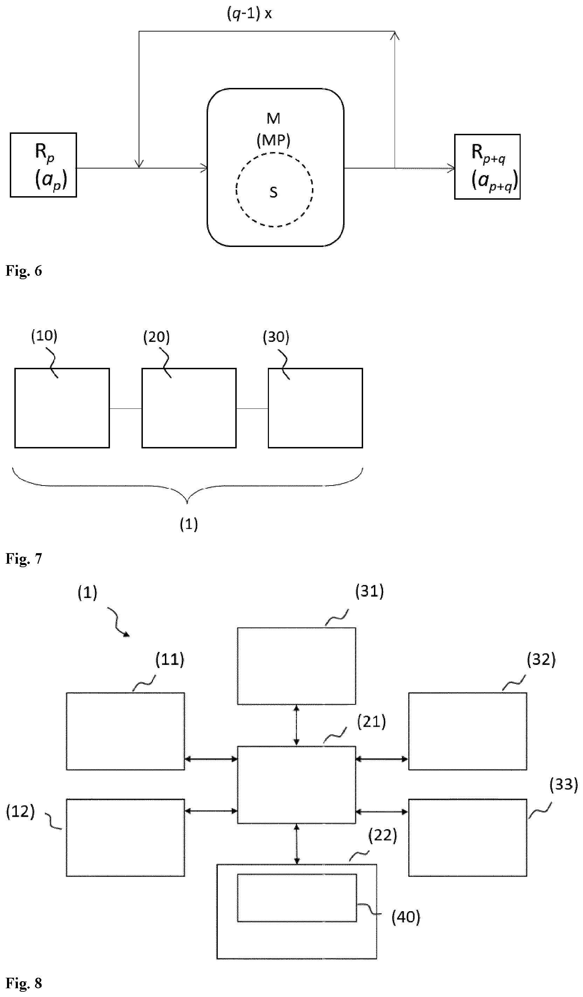

Fig. 6 zeigt eine Erweiterung des in Fig. 5 gezeigten Schemas. Während in Fig. 5 auf Basis einer Repräsentation $R_p$, die den Untersuchungsbereich nach der Applikation einer Kontrastmittelmenge $a_p$ repräsentiert, nacheinander die Repräsentationen $R_{p+1}^*$, $R_{p+2}^*$, $R_{p+3}^*$ und $R_{p+4}^*$ erzeugt werden, die den Untersuchungsbereich nach der Applikation der Mengen $a_{p+1}$, $a_{p+2}$, $a_{p+3}$ und $a_{p+4}$ des Kontrastmittels repräsentieren, ist in Fig. 6 gezeigt, wie auf Basis der Repräsentation $R_p$, die den Untersuchungsbereich nach der Applikation der Kontrastmittelmenge $a_p$ repräsentiert, nacheinander die Repräsentationen $R_{p+1}^*$ bis $R_{p+q}^*$ erzeugt werden, wobei $p$ eine ganze Zahl ist, die die Werte 2 bis $n$ (oder wie beschrieben auch größere Werte als $n$) annehmen kann, und q eine ganze Zahl größer als 1 ist. In Fig. 6 kommt der iterative Charakter des Modells des maschinellen Lernens M besonders deutlich zum Ausdruck. Das Modell des maschinellen Lernens M wird, ausgehend von der Repräsentation $R_p$ q-mal angewandt, wobei (q-1)-mal die Ausgabedaten wieder zurück in das Modell des maschinellen geschleust werden.

Es sei darauf hingewiesen, dass das (trainierte oder untrainierte) Modell des maschinellen Lernens nicht auf eine komplette radiologische Aufnahme (z.B. eine MRT-Aufnahme oder einen CT-Scan oder dergleichen) angewendet werden muss. Es ist möglich, das Modell des maschinellen Lernens nur auf einen Teil einer radiologischen Aufnahme anzuwenden. Es ist beispielsweise möglich, eine radiologische Aufnahme zunächst zu segmentieren, um einen Bereich von Interesse (engl. *region of interest)* zu identifizieren/selektieren. Das Modell kann dann beispielsweise ausschließlich auf den Bereich von Interesse angewandt werden.

Ferner kann die Anwendung des Modells des maschinellen Lernens einen oder mehrere Vorverarbeitungs- und/oder Nachverarbeitungsschritte umfassen. Es ist zum Beispiel denkbar, eine empfangene Repräsentation des Untersuchungsbereichs zunächst einer oder mehreren Transformationen zu unterziehen, wie beispielsweise einer Bewegungskorrektur, einer Farbraumumwandlung, einer Normalisierung, einer Segmentierung, eine Fourier-Transformation (z.B. zur Umwandlung von einer Bildraum-Repräsentation in eine Frequenzraum-Repräsentation), eine inverse Fourier-Transformation (z.B. zur Umwandlung von einer Frequenzraum-Repräsentation in eine Bildraum-Repräsentation) und/oder dergleichen. In einem weiteren Schritt kann die transformierte Repräsentation dem Modell des maschinellen Lernens zugeführt werden, das dann eine Reihe von Iterationen (Zyklen) durchläuft, um ausgehend von der transformierten Repräsentation eine Reihe von weiteren (nachfolgenden) Repräsentationen des Untersuchungsbereich in einer Reihe von weiteren (nachfolgenden) Zuständen zu erzeugen.

Bei dem erfindungsgemäßen Modell des maschinellen Lernens kann es sich beispielsweise um ein künstliches neuronales Netzwerk handeln, oder es kann ein solches umfassen.

Ein künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen der Repräsentationen. Üblicherweise gibt es ein Eingangsneuron für jedes Pixel oder Voxel einer Repräsentation, wenn es sich bei der Repräsentation um eine Ortsraumdarstellung in Form einer Rastergrafik handelt. Es können zusätzliche Eingangsneuronen für zusätzliche Eingangswerte (z.B. Informationen zum Untersuchungsbereich, zum Untersuchungsobjekt, zu Bedingungen, die bei der Erzeugung der Repräsentation herrschten, Informationen zu dem Kontrastmittel, Informationen zu der Kontrastmittelmenge, die dem Untersuchungsobjekt appliziert worden ist, und andere/weitere) vorhanden sein.

Die Ausgangsneuronen können dazu dienen, eine vorhergesagte Repräsentation auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN) oder es umfasst ein solches.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von vollständig verbundenen Neuronen (Dense / Fully Connected Layer).

Das Trainieren des neuronalen Netzes kann beispielsweise mittels eines Backpropagation-Verfahrens durchgeführt werden. Dabei wird für das Netz eine möglichst zuverlässige Vorhersage der Dynamik des Untersuchungsbereichs von einem Zustand über mindestens einen Zwischenzustand zu einem Endzustand angestrebt. Die Qualität der Vorhersage wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich des Effekts einer zunehmenden oder abnehmenden Kontrastmittelmenge auf den Untersuchungsbereich, die verwendet werden kann, um, auf Basis einer ersten Repräsentation, die den Untersuchungsbereich nach der Applikation einer ersten Menge des Kontrastmittels repräsentiert, eine oder mehrere Repräsentationen, die den Untersuchungsbereich nach der Applikation einer anderen Menge des Kontrastmittels repräsentiert, vorherzusagen.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte Daten anwenden lässt.

Das künstliche neuronale Netzwerk kann eine Autoencoder-Architektur aufweisen; z.B. kann das künstliche neuronale Netzwerk eine Architektur wie das U-Net aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netzwerk kann ein rekurrentes neuronales Netzwerk sein oder ein solches umfassen. Als rekurrente bzw. rückgekoppelte neuronale Netzwerke bezeichnet man neuronale Netzwerke, die sich im Gegensatz zu den Feedforward-Netzen durch Verbindungen von Neuronen einer Schicht zu Neuronen derselben oder einer vorangegangenen Schicht auszeichnen. Das künstliche neuronale Netzwerk kann beispielsweise ein *Long short-term memory* (LSTM) umfassen (siehe z.B. Y. Gao et al.: Fully convolutional structured LSTM networks for joint 4D medical image segmentation, DOI: 10.1109/ISBI.2018.8363764).

Das künstliche neuronale Netz kann ein Transfomer-Netzwerk sein (siehe z.B. D. Karimi et al.: Convolution-Free Medical Image Segmentation using Transformers, arXiv:2102.13645 [eess.IV]).

Die vorliegende Erfindung kann beispielsweise zur Reduktion der Menge an Kontrastmittel in einer radiologischen Untersuchung verwendet werden.

Für jedes Kontrastmittel gibt es eine empfohlene Menge, die einem Untersuchungsobjekt für einen definierten Zweck verabreicht werden kann. Üblicherweise haben Kontrastmittel eine Zulassung für einen definierten Zweck, wobei die Zulassung eine Information über die zu verabreichende (zu applizierende) Menge umfasst. Eine von einem Hersteller und/oder Vertreiber eines Kontrastmittels empfohlene Menge oder eine im Rahmen einer Zulassung vorgeschriebene Menge eines Kontrastmittels wird in dieser Beschreibung als Standardmenge bezeichnet.

So beträgt die Standardmenge von Gd-EOB-DTPA Dinatrium beispielsweise 0,025 mmol/kg Körpergewicht.

Die vorliegende Erfindung kann beispielsweise verwendet werden, um auf Basis einer Repräsentation, die einen Untersuchungsbereich nach der Applikation einer geringeren Menge eines Kontrastmittels als der Standardmenge des Kontrastmittels repräsentiert, eine Repräsentation des Untersuchungsbereich nach der Applikation einer Standardmenge vorherzusagen.

Dazu kann ein Modell des maschinellen Lernens auf Basis von Trainingsdaten trainiert werden, den Einfluss einer zunehmenden Menge an Kontrastmittel auf die Repräsentation des Untersuchungsbereichs zu lernen.

Der Trainingsdatensatz kann beispielsweise neben einer Repräsentation, die den Untersuchungsbereich nach der Applikation der Standardmenge des Kontrastmittels repräsentiert, eine Mehrzahl an Repräsentationen umfassen, die den Untersuchungsbereich nach der Applikation unterschiedlicher Mengen an Kontrastmittel repräsentieren, wobei die Mengen üblicherweise kleiner sind als die Standardmenge. Es ist zum Beispiel möglich, dass die Trainingsdaten, für jedes Untersuchungsobjekt einer Vielzahl an Untersuchungsobjekten, eine oder mehrere von sechs Repräsentationen umfassen, eine erste Repräsentation, eine zweite Repräsentation, eine dritte Repräsentation, eine vierte Repräsentation, eine fünfte Repräsentation und eine sechste Repräsentation.

Die erste Repräsentation kann den Untersuchungsbereich ohne Kontrastmittel repräsentieren. Die zweite Repräsentation kann den Untersuchungsbereich nach der Applikation von z.B. 20% der Standarmenge des Kontrastmittels repräsentieren. Die dritte Repräsentation kann den Untersuchungsbereich nach der Applikation von z.B. 40% der Standarmenge des Kontrastmittels repräsentieren. Die vierte Repräsentation kann den Untersuchungsbereich nach der Applikation von z.B. 60% der Standarmenge des Kontrastmittels repräsentieren. Die fünfte Repräsentation kann den Untersuchungsbereich nach der Applikation von z.B. 80% der Standarmenge des Kontrastmittels repräsentieren. Die sechste Repräsentation kann den Untersuchungsbereich nach der Applikation von z.B. 100% der Standarmenge des Kontrastmittels repräsentieren.

Das Modell des maschinellen Lernens kann auf Basis der Trainingsdaten trainiert werden, ausgehend von einer Repräsentation, die den Untersuchungsbereich nach der Applikation einer Menge des Kontrastmittels (die auch Null sein kann) repräsentiert, durch ein- oder mehrfaches Anwenden des Modells des maschinellen Lernens eine andere Repräsentation vorherzusagen, die den Untersuchungsbereich nach der Applikation einer anderen Menge des Kontrastmittels repräsentiert.

Ist das Modell trainiert, so kann es zur Vorhersage genutzt werden. Dazu kann dem trainierten Modell des maschinellen Lernens eine Repräsentation des Untersuchungsbereichs (eines anderen, nicht im Rahmen des Trainings betrachteten Untersuchungsobjekts) zugeführt werden, die den Untersuchungsbereichs nach der Applikation einer Menge des Kontrastmittels repräsentiert, die 0% oder 20% oder 40% oder 60% oder 80% der Standardmenge des Kontrastmittels repräsentiert und mit Hilfe des Modells kann durch fünffaches (im Fall von 0%), vierfaches (im Fall von 20%), dreifaches (im Fall von 40%), zweifaches (im Fall von 60%) oder einfaches (im Fall von 80%) Anwenden des trainierten Modells des maschinellen Lernens eine Repräsentation des Untersuchungsbereichs vorhergesagt werden, die den Untersuchungsbereich nach der Applikation der Standarmenge des Kontrastmittels repräsentiert.

In einem anderen Anwendungsbeispiel kann die vorliegende Erfindung verwendet werden, um eine Repräsentation vorherzsagen, die den Untersuchungsbereich nach der Applikation einer größeren Menge als der Standardmenge repräsentiert, ohne dass in dem Training eine so große Menge einem Untersuchungsobjekt appliziert werden muss.

Um bei dem zuvor genannten Beispiel zu bleiben, kann das trainierte Modell des maschinellen Lernens wiederholt angewendet werden, um eine Repräsentation des Untersuchungsbereichs vorherzusagen, die den Untersuchungsbereich nach der Applikation von 120% oder 140% oder 160% oder 180% oder 200% der Standardmenge repräsentiert. Auch Werte jenseits von 200% sind möglich. Auch wenn eine Verifikation, dass eine vorhergesagte Repräsentation im Realfall tatsächlich so aussehen würde, ausbleiben würde, weil eine Applikation so großer Mengen aus ethischen Gründen nicht vertretbar sein könnte, kann eine solche vorhergesagte Repräsentation einen Nutzen haben, wenn ein Radiologe in solchen Aufnahmen Funktionsstörungen und/oder Läsionen und/oder dergleichen erkennt, die er in anderen Aufnahmen nicht erkennen oder weniger sicher erkennen würde.

In einer besonders bevorzugten Ausführungsform wird die vorliegende Erfindung genutzt, um eine künstliche Kontrastverstärkung in einer CT-Aufnahme zu erzielen, die unter Verwendung eines MR-Kontrastmittels erzeugt worden ist.

MR-Kontrastmittel können grundsätzlich auch in der Computertomographie verwendet werden; allerdings ist die Kontrastverstärkung in der Computertomographie geringer als in der Magnetresonanztomographie, da der Kontrast von MR-Kontrastmitteln in der MRT auf einer anderen physikalischen Wirkung beruht als der Kontrast von MR-Kontrastmitteln in der CT. MR-Kontrastmittel führen zudem in der CT zu einer geringeren Kontrastverstärkung als übliche CT-Kontrastmittel, da sie eine geringere Röntgenabsorption aufweisen. Trotzdem kann es von Interesse sein, ein MR-Kontrastmittel in der CT einzusetzen, da es MR-Kontrastmittel gibt, die spezifisch von bestimmten Körperzellen aufgenommen werden oder spezifisch an bestimmte Körperzellen binden, während es keine vergleichbaren CT-Kontrastmittel gibt. Durch den Einsatz eines MR-Kontrastmittels in der CT können also Dinge sichtbar gemacht werden, die vorher in der CT nicht oder nicht sicher erkennbar waren.

Für die Magnetresonanztomographie stehen beispielsweise intrazelluläre Kontrastmittel zur Verfügung, die spezifisch von bestimmten Körperzellen aufgenommen werden. Als Beispiel sei das Dinatriumsalz der Gadoxetsäure (Gd-EOB-DTPA Dinatrium) genannt, das in dem US-Patent No. 6,039,931A beschrieben ist unter dem Markennamen Primovist® und Eovist® kommerziell erhältlich ist. Es handelt sich bei diesem Kontrastmittel um ein so genanntes hepatobiliäres Kontrastmittel, das spezifisch von Leberzellen, den Hepatozyten, aufgenommen wird, sich im Funktionsgewebe (Parenchym) anreichert und den Kontrast in gesundem Lebergewebe verstärkt. Es ist für die Verwendung in der Magnetresonanztomographie zugelassen. Für die Computertomographie ist kein vergleichbares hepatobiliäres Kontrastmittel bekannt. Die gilt auch für Gadofosveset, einem intravaskulären MR-Kontrastmittel auf Gadoliniumbasis, das an Serumalbumin im Blut bindet und damit zu einer langen Verweildauer des Kontrastmittels im Blutkreislauf führt (Halbwertszeit im Blut etwa 17 Stunden). Ein weiteres Beispiel eines intravaskulären Kontrastmittels, das in der Magnetresonanztomographie Verwendung findet, für das es aber in der Computertomographie kein vergleichbares Äquivalent gibt, ist Ferumoxytol, ein kolloidaler Eisen-Kohlenhydrat-Komplex. Ferumoxytol kann als intravenöse Injektion verabreicht werden und ist als Lösung zur intravenösen Injektion unter dem Markennamen Rienso® oder Ferahme® im Handel erhältlich. Der Eisen-Kohlenhydrat-Komplex weist superparamagnetische Eigenschaften auf und kann daher (off-label) zur Kontrastverstärkung bei MR-Untersuchungen eingesetzt werden (siehe z.B.: L.P. Smits et al.: Evaluation of ultrasmall superparamagnetic iron-oxide (USPIO) enhanced MRI with ferumoxytol to quantify arterial wall inflammation, Atherosclerosis 2017, 263: 211-218).

Es kann ein Modell des maschinellen Lernens trainiert werden, den Effekt zunehmender Mengen eines MR-Kontrastmittels auf eine Repräsentation eines Untersuchungsbereichs in einer computertomographischen Untersuchung zu lernen.

Dem Modell des maschinellen Lernens können Repräsentationen des Untersuchungsbereichs einer Vielzahl von Untersuchungsobjekten präsentiert werden, die den Untersuchungsbereich in einer CT-Untersuchung nach der Applikation unterschiedlicher Mengen eines MR-Kontrastmittels repräsentieren. Dabei kann die maximale Menge, die appliziert worden ist, beispielsweise die Standardmenge des MR-Kontrastmittels sein, wie sie für seine Verwendung in der MRT empfohlen oder zugelassen ist.

Ist das Modell des maschinellen Lernens trainiert, können Repräsentationen vorhergesagt werden, die den Untersuchungsbereich in einer CT-Untersuchung nach der Applikation einer größeren Menge des MR-Kontrastmittels als der Standardmenge repräsentieren.

Die vorliegende Erfindung kann ganz oder teilweise mit Hilfe eines Computersystems ausgeführt werden.

Fig. 7 zeigt beispielhaft und schematisch ein solches Computersystem.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 7 gezeigte Computersystem (1) umfasst eine Eingabeeinheit (10), eine Steuer- und Recheneinheit (20) und eine Ausgabeeinheit (30).

Die Steuer- und Recheneinheit (20) dient der Steuerung des Computersystems (1), der Koordinierung der Datenflüsse zwischen den Einheiten des Computersystems (1) und der Durchführung von Berechnungen.

Die Steuer- und Recheneinheit (20) ist konfiguriert

- die Eingabeeinheit (10) zu veranlassen, eine Repräsentation $R_p$ eines Untersuchungsbereichs eines Untersuchungsobjekts zu empfangen, wobei die Repräsentation $R_p$ den Untersuchungsbereich ohne Kontrastmittel oder nach einer Applikation einer Menge $a_p$ eines Kontrastmittels repräsentiert, wobei $p$ eine ganze Zahl ist, die kleiner als $n$ ist, wobei $n$ eine ganze Zahl größer als 2 ist,

- die Repräsentation $R_p$ einem trainierten Modell des maschinellen Lernens zuzuführen, wobei das Modell des maschinellen Lernens auf Basis von Trainingsdaten trainiert worden ist, ausgehend von einer ersten Repräsentation $^{T}R_1$ nacheinander eine Zahl $n-1$ von Repräsentationen $^{T}R_2{}^*$ bis $^{T}R_n{}^*$ zu erzeugen, wobei die erste Repräsentation $^{T}R_1$ den Untersuchungsbereich ohne Kontrastmittel oder nach Applikation einer ersten Menge $a_1$ des Kontrastmittels repräsentiert und jede erzeugte Repräsentation $^{T}R_j{}^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^{T}R_2{}^*$ zumindest anteilig auf Basis der Repräsentation $^{T}R_1$ erfolgt und die Erzeugung jeder weiteren Repräsentation $^{T}R_k{}^*$ zumindest anteilig auf Basis der jeweils zuvor erzeugten Repräsentation $^{T}R_{k-1}{}^*$ erfolgt, wobei $k$ ein Index ist, der ganze Zahlen von 3 bis $n$ durchläuft,

- von dem Modell des maschinellen Lernens eine oder mehrere Repräsentationen $R_{p+q}{}^*$ des Untersuchungsbereichs zu empfangen, wobei jede der einen oder der mehreren Repräsentationen $R_{p+q}{}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n-1$ ist oder gleich $n-1$ ist oder größer als $n-1$ ist,

- die Ausgabeeinheit (30) zu veranlassen, die eine oder die mehreren Repräsentationen $R_{p+q}{}^*$ zu speichern und/oder auszugeben und/oder an ein separates Computersystem zu übermitteln.

Fig. 8 zeigt beispielhaft und schematisch eine weitere Ausführungsform des erfindungsgemäßen Computersystems. Das Computersystem (1) umfasst eine Verarbeitungseinheit (21), die mit einem Speicher (22) verbunden ist. Die Verarbeitungseinheit (21) und der Speicher (22) bilden eine Steuer-und Recheneinheit, wie sie in Fig. 7 gezeigt ist.

Die Verarbeitungseinheit (21) (engl.: *processing unit*) kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen (z.B. Repräsentation des Untersuchungsbereichs), Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (32, 33) und/oder eine oder mehrere Benutzerschnittstellen (11, 12, 31) umfassen. Die eine oder mehrere Kommunikationsschnittstellen (32, 33) können so konfiguriert sein, dass sie Informationen senden und/oder empfangen, z.B. zu und/oder von einem MRT-Scanner, einem CT-Scanner, einer Ultraschallkamera, anderen Computersystemen, Netzwerken, Datenspeichern oder dergleichen. Die eine oder mehrere Kommunikationsschnittstellen (32, 33) können so konfiguriert sein, dass sie Informationen über physische (verdrahtete) und/oder drahtlose Kommunikationsverbindungen übertragen und/oder empfangen. Die eine oder die mehreren Kommunikationsschnittstellen (32, 33) können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen (32, 33) eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen (11, 12, 31) können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen (11, 12) sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen (11, 12, 31) eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen (11, 12, 31) können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

In dem Speicher (22) kann auch das erfindungsgemäße Modell des maschinellen Lernens gespeichert sein.

Das Computersystem der vorliegenden Offenbarung kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

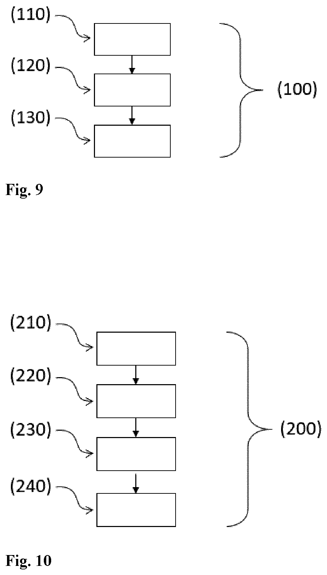

Fig. 9 zeigt schematisch in Form eines Ablaufschemas eine Ausführungsform des erfindungsgemäßen Trainingsverfahrens. Das Trainingsverfahren (100) umfasst die Schritte:

(110) Empfangen von Trainingsdaten,

- wobei die Trainingsdaten für eine Vielzahl von Untersuchungsobjekten Repräsentationen ${}^{T}R_1$ bis ${}^{T}R_n$ eines Untersuchungsbereichs umfassen, wobei $n$ eine ganze Zahl ist, die größer als zwei ist,

- wobei jede Repräsentation ${}^{T}R_1$ den Untersuchungsbereich nach Applikation einer Menge $a_i$ eines Kontrastmittels repräsentiert, wobei $i$ eine ganze Zahl im Bereich von 1 bis $n$ ist, wobei die Mengen $a_1$ bis $a_n$ eine Folge von vorzugsweise zunehmenden Mengen bilden,

(120) Trainieren des Modells des maschinellen Lernens,

- wobei das Modell trainiert wird, ausgehend von der Repräsentation ${}^{T}R_1$ nacheinander Repräsentationen ${}^{T}R_2^*$ bis ${}^{T}R_n^*$ zu erzeugen,

- wobei die Erzeugung der Repräsentation ${}^{T}R_2^*$ zumindest anteilig auf Basis der Repräsentation ${}^{T}R_1$ erfolgt und die Erzeugung jeder weiteren Repräsentation ${}^{T}R_k^*$ zumindest anteilig auf Basis der jeweils zuvor erzeugten Repräsentation ${}^{T}R_{k-1}^*$ erfolgt, wobei $k$ ein Index ist, der ganze Zahlen von 3 bis $n$ durchläuft, wobei die Repräsentation ${}^{T}R_k^*$ den Untersuchungsbereich nach Applikation der Menge $a_k$ des Kontrastmittels repräsentiert und die Repräsentation ${}^{T}R_{k-1}^*$ den Untersuchungsbereich nach Applikation der Menge $a_{k-1}$ des Kontrastmittels repräsentiert,

- wobei mittels einer Fehlerfunktion Abweichungen zwischen den erzeugten Repräsentationen ${}^{T}R_j^*$ und den Repräsentationen ${}^{T}R_1$ quantifiziert werden, wobei $j$ ein Index ist, der die Zahlen von 2 bis $n$ durchläuft,

- wobei die Abweichungen durch Modifizieren von Modellparametern des Modells des maschinellen Lernens in einem Optimierungsverfahren minimiert werden,

(130) Verwenden des Modells des maschinellen Lernens zur Vorhersage.

Fig. 10 zeigt schematisch in Form eines Ablaufschemas eine Ausführungsform des erfindungsgemäßen Vorhersageverfahrens. Das Vorhersageverfahren (200) umfasst die Schritte:

(210) Empfangen einer Repräsentation $R_p$ des Untersuchungsbereichs,

- wobei die Repräsentation $R_p$ den Untersuchungsbereich ohne Kontrastmittel oder nach Applikation einer Menge $a_p$ eines Kontrastmittels repräsentiert, wobei $p$ eine ganze Zahl ist, die kleiner als $n$ ist, wobei $n$ eine ganze Zahl größer als 2 ist,

(220) Zuführen der Repräsentation $R_p$ einem trainierten Modell des maschinellen Lernens,

- wobei das Modell des maschinellen Lernens auf Basis von Trainingsdaten trainiert worden ist, ausgehend von einer ersten Repräsentation $^TR_1$ nacheinander eine Zahl $n-1$ von Repräsentationen $^TR_2^*$ bis $^TR_n^*$ zu erzeugen,
- wobei die erste Repräsentation $^TR_1$ den Untersuchungsbereich ohne Kontrastmittel oder nach Applikation einer ersten Menge $a_1$ des Kontrastmittels repräsentiert und jede erzeugte Repräsentation $^TR_j^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden,
- wobei die Erzeugung der Repräsentation $^TR_2^*$ zumindest anteilig auf Basis der Repräsentation $^TR_1$ erfolgt und die Erzeugung jeder weiteren Repräsentation $^TR_k^*$ zumindest anteilig auf Basis der jeweils zuvor erzeugten Repräsentation $^TR_{k-1}^*$ erfolgt, wobei $k$ ein Index ist, der ganze Zahlen von 3 bis $n$ durchläuft,

(230) Empfangen einer oder mehrerer Repräsentationen $R_{p+q}^*$ des Untersuchungsbereichs von dem Modell des maschinellen Lernens,

- wobei jede der einen oder der mehreren Repräsentationen $R_{p+q}^*$ den Untersuchungsbereich nach der Applikation der Menge $a_{p+q}$ des Kontrastmittels repräsentiert, wobei $q$ ein Index ist, der die Zahlen von 1 bis $m$ durchläuft, wobei $m$ eine ganze Zahl ist, die kleiner als $n-1$ ist oder gleich $n-1$ ist oder größer als $n-1$ ist,

(240) Ausgeben und/oder Speichern und/oder Übermitteln der einen oder der mehreren Repräsentationen $R_{p+q}^*$.

**Patentansprüche**

1. Computer-implementiertes Verfahren zum Trainieren und Verwenden eines Modells (M) des maschinellen Lernens, wobei das Verfahren umfasst:

   - Empfangen von Trainingsdaten,

     • wobei die Trainingsdaten für eine Vielzahl von Untersuchsobjekten Repräsentationen $^TR_1$ bis $^TR_n$ eines Untersuchungsbereichs umfassen, wobei $n$ eine ganze Zahl ist, die größer als zwei ist,
     • wobei jede Repräsentation $^TR_i$ den Untersuchungsbereich nach Applikation einer Menge $a_i$ eines Kontrastmittels repräsentiert, wobei $i$ eine ganze Zahl im Bereich von 1 bis $n$ ist, wobei die Mengen $a_1$ bis $a_n$ eine Folge von vorzugsweise zunehmenden Mengen bilden,
     • wobei jedes Untersuchungsobjekt vorzugsweise ein Mensch ist und der Untersuchungsbereich vorzugsweise ein Teil des Menschen ist,

   - Trainieren des Modells (M) des maschinellen Lernens,

     • wobei das Modell (M) trainiert wird, ausgehend von der Repräsentation $^TR_1$ nacheinander Repräsentationen $^TR_2^*$ bis $^TR_n^*$ zu erzeugen,
     • wobei die Erzeugung der Repräsentation $^TR_2^*$ zumindest anteilig auf Basis der Repräsentation $^TR_1$ erfolgt und die Erzeugung jeder weiteren Repräsentation $^TR_k^*$ zumindest anteilig auf Basis der jeweils zuvor erzeugten Repräsentation $^TR_{k-1}^*$ erfolgt, wobei $k$ ein Index ist, der ganze Zahlen von 3 bis $n$ durchläuft, wobei die Repräsentation $^TR_k^*$ den Untersuchungsbereich nach Applikation der Menge $a_k$ des Kontrastmittels repräsentiert und die Repräsentation $^TR_{k-1}^*$ den Untersuchungsbereich nach Applikation der Menge $a_{k-1}$ des Kontrastmittels repräsentiert,
     • wobei mittels einer Fehlerfunktion Abweichungen zwischen den erzeugten Repräsentationen $^TR_j^*$ und den Repräsentationen $^TR_j$ quantifiziert werden, wobei $j$ ein Index ist, der die Zahlen von 2 bis $n$ durchläuft,
     • wobei die Abweichungen durch Modifizieren von Modellparametern (MP) des Modells (M) des maschinellen Lernens minimiert werden,

   - Verwenden des Modells (M) des maschinellen Lernens zur Vorhersage.

2. Verfahren gemäß Anspruch 1, ferner umfassend die Schritte:

- für jede erzeugte Repräsentation $^TR_j^*$: Berechnen eines Fehlerwerts für jedes Paar aus einer Repräsentation $^TR_j$ und der erzeugten Repräsentationen $^TR_j^*$ mit Hilfe einer Fehlerfunktion, wobei $j$ ein Index ist, der die Zahlen von 2 bis $n$ durchläuft,
- Berechnen eines Gesamtfehlerwerts mit Hilfe einer Gesamtfehlerfunktion, wobei die Gesamtfehlerfunktion eine Funktion der Fehlerwerte ist,
- Minimieren des Gesamtfehlerwerts durch Modifizieren der Modellparameter (MP).

3. Verfahren gemäß Anspruch 2, wobei die Gesamtfehlerfunktion folgende Formel hat:

$$LV = \sum_{j=2}^{n} w_j \cdot LF_j$$

wobei LV der Gesamtfehlerwert ist, wobei $LF_j$ die Fehlerfunktionen für die Berechnung der Fehlerwerte für die Abweichungen zwischen der Repräsentation $^TR_j$ und der erzeugten Repräsentation $^TR_j^*$ sind, und $w_j$ Gewichtsfaktoren sind, wobei $j$ ein Index ist, der die Zahlen von 2 bis $n$ durchläuft.

4. Computer-implementiertes Verfahren zur Vorhersage einer Repräsentation eines Untersuchungsbereichs eines Untersuchungsobjekts umfassend:

- Empfangen einer Repräsentation $R_p$ des Untersuchungsbereichs,

• wobei die Repräsentation $R_p$ den Untersuchungsbereich ohne Kontrastmittel oder nach Applikation einer Menge $a_p$ eines Kontrastmittels repräsentiert, wobei $p$ eine ganze Zahl ist, die kleiner als $n$ ist, wobei $n$ eine ganze Zahl größer als 2 ist, wobei das Untersuchungsobjekt vorzugsweise ein Mensch ist und der Untersuchungsbereich vorzugsweise ein Teil des Menschen ist,

- Zuführen der Repräsentation $R_p$ einem trainierten Modell (M) des maschinellen Lernens,

• wobei das Modell (M) des maschinellen Lernens auf Basis von Trainingsdaten trainiert worden ist, ausgehend von einer ersten Repräsentation $^TR_1$ nacheinander eine Zahl $n$-1 von Repräsentationen $^TR_2^*$ bis $^TR_n^*$ zu erzeugen,
• wobei die erste Repräsentation $^TR_1$ den Untersuchungsbereich ohne Kontrastmittel oder nach Applikation einer ersten Menge $a_1$ des Kontrastmittels repräsentiert und jede erzeugte Repräsentation $^TR_j^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden,
• wobei die Erzeugung der Repräsentation $^TR_2^*$ zumindest anteilig auf Basis der Repräsentation $^TR_1$ erfolgt und die Erzeugung jeder weiteren Repräsentation $^TR_k^*$ zumindest anteilig auf Basis der jeweils zuvor erzeugten Repräsentation $^TR_{k-1}^*$ erfolgt, wobei $k$ ein Index ist, der ganze Zahlen von 3 bis $n$ durchläuft,

- Empfangen einer oder mehrerer Repräsentationen $R_{p+q}^*$ des Untersuchungsbereichs von dem Modell (M) des maschinellen Lernens,

• wobei jede der einen oder der mehreren Repräsentationen $R_{p+q}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n$-1 ist oder gleich $n$-1 ist oder größer als $n$-1 ist,

- Ausgeben und/oder Speichern und/oder Übermitteln der einen oder der mehreren Repräsentationen $R_{p+q}^*$.

5. Verfahren gemäß Anspruch 4, wobei das Verfahren umfasst:

- Empfangen der Repräsentation $R_p$ des Untersuchungsbereichs, wobei die Repräsentation $R_p$ den Untersuchungsbereich ohne Kontrastmittel oder nach Applikation der Menge $a_p$ des Kontrastmittels repräsentiert, wobei $p$ eine ganze Zahl ist, die kleiner als $n$ ist,
- Zuführen der Repräsentation $R_p$ dem trainierten Modell (M) des maschinellen Lernens, wobei das Modell (M) des maschinellen Lernens in einem Verfahren gemäß einem der Ansprüche 1 bis 3 trainiert worden ist,

- Empfangen der einen oder mehreren Repräsentationen $R_{p+q}^*$ des Untersuchungsbereichs von dem Modell (M) des maschinellen Lernens, wobei jede der einen oder der mehreren Repräsentationen $R_{p+q}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n$-1 ist oder gleich $n$-1 ist oder größer als $n$-1 ist,
- Ausgeben und/oder Speichern und/oder Übermitteln der einen oder der mehreren Repräsentationen $R_{p+q}^*$.

6. Verfahren gemäß einem der Ansprüche 1 bis 5, wobei jede von dem Modell (M) des maschinellen Lernens erzeugte Repräsentation $^TR_{p+q}^*$ und/oder $R_{p+q}^*$ wieder dem trainierten Modell (M) des maschinellen Lernens zugeführt wird, um eine Repräsentation $^TR_{p+q+1}^*$ und/oder $R_{p+q+1}^*$ zu erzeugen.

7. Verfahren gemäß einem der Ansprüche 4 bis 6, wobei $m$ im Bereich von $n$ bis $n$+10 liegt.

8. Verfahren gemäß einem der Ansprüche 1 bis 7, wobei jede erzeugte Repräsentation nach der folgenden Formel berechnet wird:

$$M^q\,(R_1) = R_{1+q}{}^*$$

wobei M eine Transformation repräsentiert, die auf Eingabedaten des Modell (M) des maschinellen Lernens durch das Modell (M) des maschinellen Lernens angewandt wird, wobei $M^q$ die q-fache Anwendung der Transformation bedeutet, wobei in ein einem ersten Schritt die Transformation auf eine erste Repräsentation $R_1$ angewendet wird, in einem zweiten Schritt die Transformation auf das Ergebnis der Anwendung im ersten Schritt angewandt wird, indem das Ergebnis zurück in das Modell (M) des maschinellen Lernens geführt wird und der Vorgang mit jedem weiteren Ergebnis einer Transformation wiederholt wird, bis die Transformation insgesamt q-mal angewandt worden ist, wobei q eine ganze Zahl ist, die die Werte 1 bis $m$ annehmen kann, wobei $m$ eine ganze Zahl ist, die kleiner als $n$-1 ist oder gleich $n$-1 ist oder größer als $n$-1 ist.

9. Verfahren gemäß einem der Ansprüche 1 bis 8, wobei der Untersuchungsbereich die menschliche Leber ist oder einen Teil der menschlichen Leber ist.

10. Verfahren gemäß einem der Ansprüche 1 bis 9, wobei es sich bei den empfangenen Repräsentationen um CT-Aufnahmen, MRT-Aufnahmen oder Ultraschallaufnahmen handelt.

11. Verfahren gemäß einem der Ansprüche 1 bis 9, wobei es sich bei den empfangenen Repräsentationen um CT-Aufnahmen handelt und wobei es sich bei dem Kontrastmittel um ein MR-Kontrastmittel handelt.

12. Computersystem (1) umfassend

• eine Eingabeeinheit (10),
• eine Steuer- und Recheneinheit (20) und
• eine Ausgabeeinheit (30),

wobei die Steuer- und Recheneinheit (20) konfiguriert ist,

- die Eingabeeinheit (10) zu veranlassen, eine Repräsentation $R_p$ eines Untersuchungsbereichs eines Untersuchungsobjekts zu empfangen, wobei die Repräsentation $R_p$ den Untersuchungsbereich ohne Kontrastmittel oder nach einer Applikation einer Menge $a_p$ eines Kontrastmittels repräsentiert, wobei p eine ganze Zahl ist, die kleiner als $n$ ist, wobei $n$ eine ganze Zahl größer als 2 ist, wobei das Untersuchungsobjekt vorzugsweise ein Mensch ist und der Untersuchungsbereich vorzugsweise ein Teil des Menschen ist,
- die Repräsentation $R_p$ einem trainierten Modell (M) des maschinellen Lernens zuzuführen, wobei das Modell (M) des maschinellen Lernens auf Basis von Trainingsdaten trainiert worden ist, ausgehend von einer ersten Repräsentation $^TR_1$ nacheinander eine Zahl $n$-1 von Repräsentationen $^TR_2^*$ bis $^TR_n^*$ zu erzeugen, wobei die erste Repräsentation $^TR_1$ den Untersuchungsbereich ohne Kontrastmittel oder nach Applikation einer ersten Menge $a_1$ des Kontrastmittels repräsentiert und jede erzeugte Repräsentation $^TR_j^*$ den Untersuchungsbereich nach Applikation einer Menge $a_i$ des Kontrastmittels repräsentiert, wobei j ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^TR_2^*$ zumindest anteilig auf Basis der Repräsentation $^TR_1$ erfolgt und die

Erzeugung jeder weiteren Repräsentation $^TR_k^*$ zumindest anteilig auf Basis der jeweils zuvor erzeugten Repräsentation $^TR_{k-1}^*$ erfolgt, wobei $k$ ein Index ist, der ganze Zahlen von 3 bis $n$ durchläuft,

- von dem Modell (M) des maschinellen Lernens eine oder mehrere Repräsentationen $R_{p+q}^*$ des Untersuchungsbereichs zu empfangen, wobei jede der einen oder der mehreren Repräsentationen $R_{p+q}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n-1$ ist oder gleich $n-1$ ist oder größer als $n-1$ ist,

- die Ausgabeeinheit (30) zu veranlassen, die eine oder die mehreren Repräsentationen $R_{p+q}^*$ zu speichern und/oder auszugeben und/oder an ein separates Computersystem zu übermitteln.

**13.** Computerprogrammprodukt umfassend ein Computerprogramm (40), das in einen Arbeitsspeicher (22) eines Computersystems (1) geladen werden kann und dort das Computersystem (1) dazu veranlasst, folgende Schritte ausführen:

- Empfangen einer Repräsentation $R_p$ eines Untersuchungsbereichs eines Untersuchungsobjekts, wobei die Repräsentation $R_p$ den Untersuchungsbereich ohne Kontrastmittel oder nach Applikation einer Menge $a_p$ eines Kontrastmittels repräsentiert, wobei $p$ eine ganze Zahl ist, die kleiner als $n$ ist, wobei $n$ eine ganze Zahl größer als 2 ist, wobei das Untersuchungsobjekt vorzugsweise ein Mensch ist und der Untersuchungsbereich vorzugsweise ein Teil des Menschen ist,

- Zuführen der Repräsentation $R_p$ einem trainierten Modell (M) des maschinellen Lernens, wobei das Modell (M) des maschinellen Lernens auf Basis von Trainingsdaten trainiert worden ist, ausgehend von einer ersten Repräsentation $^TR_1$ nacheinander eine Zahl $n-1$ von Repräsentationen $^TR_2^*$ bis $^TR_n^*$ zu erzeugen, wobei die erste Repräsentation $^TR_1$ den Untersuchungsbereich ohne Kontrastmittel oder nach Applikation einer ersten Menge $a_1$ des Kontrastmittels repräsentiert und jede erzeugte Repräsentation $^TR_j^*$ den Untersuchungsbereich nach einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^TR_2^*$ zumindest anteilig auf Basis der Repräsentation $^TR_1$ erfolgt und die Erzeugung jeder weiteren Repräsentation $^TR_k^*$ zumindest anteilig auf Basis der jeweils zuvor erzeugten Repräsentation $^TR_{k-1}^*$ erfolgt, wobei $k$ ein Index ist, der ganze Zahlen von 3 bis $n$ durchläuft,

- Empfangen einer oder mehrerer Repräsentationen $R_{p+q}^*$ des Untersuchungsbereichs von dem Modell (M) des maschinellen Lernens, wobei jede der einen oder der mehreren Repräsentationen $R_{p+q}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n-1$ ist oder gleich $n-1$ ist oder größer als $n-1$ ist,

- Ausgeben und/oder Speichern und/oder Übermitteln der einen oder der mehreren Repräsentationen $R_{p+q}^*$.

**14.** Verwendung eines Kontrastmittels in einem radiologischen Untersuchungsverfahren, wobei das radiologische Untersuchungsverfahren die folgenden Schritte umfasst:

- Empfangen einer Repräsentation $R_p$ eines Untersuchungsbereichs eines Untersuchungsobjekts, wobei die Repräsentation $R_p$ den Untersuchungsbereich ohne Kontrastmittel oder nach Applikation einer Menge $a_p$ des Kontrastmittels repräsentiert, wobei $p$ eine ganze Zahl ist, die kleiner als $n$ ist, wobei $n$ eine ganze Zahl größer als 2 ist,

- Zuführen der Repräsentation $R_p$ einem trainierten Modell (M) des maschinellen Lernens, wobei das Modell (M) des maschinellen Lernens auf Basis von Trainingsdaten trainiert worden ist, ausgehend von einer ersten Repräsentation $^TR_1$ nacheinander eine Zahl $n-1$ von Repräsentationen $^TR_2^*$ bis $^TR_n^*$ zu erzeugen, wobei die erste Repräsentation $^TR_1$ den Untersuchungsbereich ohne Kontrastmittel oder nach Applikation einer ersten Menge $a_1$ des Kontrastmittels repräsentiert und jede erzeugte Repräsentation $^TR_j^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^TR_2^*$ zumindest anteilig auf Basis der Repräsentation $^TR_1$ erfolgt und die Erzeugung jeder weiteren Repräsentation $^TR_k^*$ zumindest anteilig auf Basis der jeweils zuvor erzeugten Repräsentation $^TR_{k-1}^*$ erfolgt, wobei $k$ ein Index ist, der ganze Zahlen von 3 bis $n$ durchläuft,

- Empfangen einer oder mehrerer Repräsentationen $R_{p+q}^*$ des Untersuchungsbereichs von dem Modell (M) des maschinellen Lernens, wobei jede der einen oder der mehreren Repräsentationen $R_{p+q}^*$ den Untersuchungsbereich nach der Applikation der Menge $a_{p+q}$ des Kontrastmittels repräsentiert, wobei q ein Index ist, der die Zahlen von 1 bis $m$ durchläuft, wobei $m$ eine ganze Zahl ist, die kleiner als $n-1$ ist oder gleich $n-1$ ist oder größer als $n-1$ ist,

- Ausgeben und/oder Speichern und/oder Übermitteln der einen oder der mehreren Repräsentationen $R_{p+q}{}^*$.

15. Kit umfassend ein Kontrastmittel und ein Computerprogrammprodukt, wobei das Computerprogrammprodukt ein Computerprogramm (40) umfasst, das in einen Arbeitsspeicher (22) eines Computersystems (1) geladen werden kann und dort das Computersystem (1) dazu veranlasst, folgende Schritte auszuführen:

- Empfangen einer Repräsentation $R_p$ eines Untersuchungsbereichs eines Untersuchungsobjekts, wobei die Repräsentation $R_p$ den Untersuchungsbereich ohne Kontrastmittel oder nach Applikation einer Menge $a_p$ des Kontrastmittels repräsentiert, wobei $p$ eine ganze Zahl ist, die kleiner als $n$ ist, wobei $n$ eine ganze Zahl größer als 2 ist,
- Zuführen der Repräsentation $R_p$ einem trainierten Modell (M) des maschinellen Lernens, wobei das Modell (M) des maschinellen Lernens auf Basis von Trainingsdaten trainiert worden ist, ausgehend von einer ersten Repräsentation $^TR_1$ nacheinander eine Zahl $n$-1 von Repräsentationen $^TR_2{}^*$ bis $^TR_n{}^*$ zu erzeugen, wobei die erste Repräsentation $^TR_1$ den Untersuchungsbereich ohne Kontrastmittel oder nach Applikation einer ersten Menge $a_1$ des Kontrastmittels repräsentiert und jede erzeugte Repräsentation $^TR_j{}^*$ den Untersuchungsbereich nach Applikation einer Menge $a_j$ des Kontrastmittels repräsentiert, wobei $j$ ein Index ist, der ganze Zahlen von 2 bis $n$ durchläuft, wobei die Mengen $a_1$ bis $a_n$ eine Folge von vorzugsweise zunehmenden Mengen bilden, wobei die Erzeugung der Repräsentation $^TR_2{}^*$ zumindest anteilig auf Basis der Repräsentation $^TR_1$ erfolgt und die Erzeugung jeder weiteren Repräsentation $^TR_k{}^*$ zumindest anteilig auf Basis der jeweils zuvor erzeugten Repräsentation $^TR_{k-1}{}^*$ erfolgt, wobei $k$ ein Index ist, der ganze Zahlen von 3 bis $n$ durchläuft,
- Empfangen einer oder mehrerer Repräsentationen $R_{p+q}{}^*$ des Untersuchungsbereichs von dem Modell (M) des maschinellen Lernens, wobei jede der einen oder der mehreren Repräsentationen $R_{p+q}{}^*$ den Untersuchungsbereich nach der Applikation der Menge $a_{p+q}$ des Kontrastmittels repräsentiert, wobei $q$ ein Index ist, der die Zahlen von 1 bis $m$ durchläuft, wobei $m$ eine ganze Zahl ist, die kleiner als $n$-1 ist oder gleich $n$-1 ist oder größer als $n$-1 ist,
- Ausgeben und/oder Speichern und/oder Übermitteln der einen oder der mehreren Repräsentationen $R_{p+q}{}^*$.

**Claims**

1. Computer-implemented method for training and using a machine-learning model (M), wherein the method comprises:

- receiving training data,

• where the training data comprise representations $^TR_1$ to $^TR_n$ of an examination region for a multiplicity of examination objects, where $n$ is an integer greater than two,
• where each representation $^TR_i$ represents the examination region after administration of an amount $a_i$ of a contrast agent, where $i$ is an integer in the range from 1 to $n$, where the amounts $a_1$ to $a_n$ form a sequence of preferably increasing amounts,
• where each examination object is preferably a human and the examination region is preferably part of the human,

- training the machine-learning model (M),

• where the model (M) is trained to generate, starting from the representation $^TR_1$, representations $^TR_2{}^*$ to $^TR_n{}^*$ one after the other,
• where the representation $^TR_2{}^*$ is generated at least partly on the basis of the representation $^TR_1$ and each further representation $^TR_k{}^*$ is generated at least partly on the basis of the respective previously generated representation $^TR_{k-1}{}^*$, where $k$ is an index passing through integers from 3 to $n$, where the representation $^TR_k{}^*$ represents the examination region after administration of the amount $a_k$ of the contrast agent and the representation $^TR_{k-1}{}^*$ represents the examination region after administration of the amount $a_{k-1}$ of the contrast agent,
• where differences between the generated representations $^TR_j{}^*$ and the representations $^TR_j$ are quantified by means of a loss function, where $j$ is an index passing through the numbers from 2 to $n$,
• where the differences are minimized by modifying model parameters (MP) of the machine-learning model (M),

- using the machine-learning model (M) for prediction.

**2.** Method according to Claim 1, further comprising the steps of:

- for each generated representation $^T\!R_j^*$: calculating a loss value for each pair composed of a representation $^T\!R_j$ and the generated representation $^T\!R_j^*$ with the aid of a loss function, where $j$ is an index passing through the numbers from 2 to $n$,
- calculating a total loss value with the aid of a total loss function, where the total loss function is a function of the loss values,
- minimizing the total loss value by modifying the model parameters (MP).

**3.** Method according to Claim 2, wherein the total loss function has the following formula:

$$\mathrm{LV} \;=\; \sum_{j=2}^{n} \mathrm{w}_j \cdot \mathrm{LF}_j$$

where LV is the total loss value, where $\mathrm{LF}_j$ are the loss functions for calculating the loss values for the differences between the representation $^T\!R_j$ and the generated representation $^T\!R_j^*$, and $\mathrm{w}_j$ are weight factors, where $j$ is an index passing through the numbers from 2 to $n$.

**4.** Computer-implemented method for predicting a representation of an examination region of an examination object, comprising:

- receiving a representation $R_p$ of the examination region,

  • where the representation $R_p$ represents the examination region without contrast agent or after administration of an amount $a_p$ of a contrast agent, where $p$ is an integer less than $n$, where $n$ is an integer greater than 2, where the examination object is preferably a human and the examination region is preferably part of the human,

- feeding the representation $R_p$ to a trained machine-learning model (M),

  • where the machine-learning model (M) has been trained on the basis of training data to generate, starting from a first representation $^T\!R_1$, a number $n$-1 of representations $^T\!R_2^*$ to $^T\!R_n^*$ one after the other,
  • where the first representation $^T\!R_1$ represents the examination region without contrast agent or after administration of a first amount $a_1$ of the contrast agent and each generated representation $^T\!R_j^*$ represents the examination region after administration of an amount $a_j$ of the contrast agent, where $j$ is an index passing through integers from 2 to $n$, where the amounts $a_1$ to $a_n$ form a sequence of preferably increasing amounts,
  • where the representation $^T\!R_2^*$ is generated at least partly on the basis of the representation $^T\!R_1$ and each further representation $^T\!R_k^*$ is generated at least partly on the basis of the respective previously generated representation $^T\!R_{k-1}^*$, where $k$ is an index passing through integers from 3 to $n$,

- receiving one or more representations $R_{p+q}^*$ of the examination region from the machine-learning model (M),

  • where each of the one or more representations $R_{p+q}^*$ represents the examination region after administration of the amount $a_{p+q}$ of the contrast agent, where $q$ is an index passing through the numbers from 1 to $m$, where $m$ is an integer less than $n$-1 or equal to $n$-1 or greater than $n$-1,

- outputting and/or storing and/or transmitting the one or more representations $R_{p+q}^*$.

**5.** Method according to Claim 4, wherein the method comprises:

- receiving the representation $R_p$ of the examination region, where the representation $R_p$ represents the examination region without contrast agent or after administration of the amount $a_p$ of the contrast agent, where $p$ is an integer less than $n$,
- feeding the representation $R_p$ to the trained machine-learning model (M), where the machine-learning model (M) has been trained in a method according to any of Claims 1 to 3,
- receiving the one or more representations $R_{p+q}^*$ of the examination region from the machine-learning model (M), where each of the one or more representations $R_{p+q}^*$ represents the examination region after administration of the amount $a_{p+q}$ of the contrast agent, where $q$ is an index passing through the numbers from 1 to $m$, where m is an integer less than $n$-1 or equal to $n$-1 or greater than $n$-1,
- outputting and/or storing and/or transmitting the one or more representations $R_{p+q}{}^*$.

6. Method according to any of Claims 1 to 5, wherein each representation $^T R_{p+q}{}^*$ and/or $R_{p+q}{}^*$ generated by the machine-learning model (M) is returned to the trained machine-learning model (M) in order to generate a representation $^T R_{p+q+1}{}^*$ and/or $R_{p+q+1}{}^*$.

7. Method according to any of Claims 4 to 6, wherein $m$ is in the range from $n$ to $n+10$.

8. Method according to any of Claims 1 to 7, wherein each generated representation is calculated according to the following formula:

$$M^q \ (R_1) \ = \ R_{1+q}{}^*$$

where M represents a transformation which is applied by the machine-learning model (M) to input data of the machine-learning model (M), where $M^q$ means the $q$-times application of the transformation, where in a first step the transformation is applied to a first representation $R_1$, in a second step the transformation is applied to the result of the application in the first step in that the result is passed back into the machine-learning model (M) and the procedure is repeated with each further result of a transformation until the transformation has been applied a total of $q$ times, where $q$ is an integer which may assume the values of 1 to $m$, where $m$ is an integer less than $n$-1 or equal to n-1 or greater than $n$-1.

9. Method according to any of Claims 1 to 8, wherein the examination region is the human liver or is part of the human liver.

10. Method according to any of Claims 1 to 9, wherein the received representations are CT images, MRI images or ultrasound images.

11. Method according to any of Claims 1 to 9, wherein the received representations are CT images and wherein the contrast agent is an MRI contrast agent.

12. Computer system (1) comprising

    • an input unit (10),
    • a control and calculation unit (20) and
    • an output unit (30),

wherein the control and calculation unit (20) is configured

    - to cause the input unit (10) to receive a representation $R_p$ of an examination region of an examination object, where the representation $R_p$ represents the examination region without contrast agent or after administration of an amount $a_p$ of a contrast agent, where $p$ is an integer less than $n$, where $n$ is an integer greater than 2, where the examination object is preferably a human and the examination region is preferably part of the human,
    - to feed the representation $R_p$ to a trained machine-learning model (M), where the machine-learning model (M) has been trained on the basis of training data to generate, starting from a first representation $^T R_1$, a number $n$-1 of representations $^T R_2{}^*$ to $^T R_n{}^*$ one after the other, where the first representation $^T R_1$ represents the examination region without contrast agent or after administration of a first amount $a_1$ of the contrast agent and each generated representation $^T R_j{}^*$ represents the examination region after administration of an amount $a_j$ of the contrast agent, where $j$ is an index passing through integers from 2 to $n$, where the amounts $a_1$ to $a_n$ form a sequence of preferably increasing amounts, where the representation $^T R_2{}^*$ is generated at least partly on the basis of the representation $^T R_1$ and each further representation $^T R_k{}^*$ is generated at least partly on the basis of the respective previously generated representation $^T R_{k-1}{}^*$, where $k$ is an index passing through integers from 3 to $n$,
    - to receive from the machine-learning model (M) one or more representations $R_{p+q}{}^*$ of the examination region, where each of the one or more representations $R_{p+q}{}^*$ represents the examination region after administration of the amount $a_{p+q}$ of the contrast agent, where $q$ is an index passing through the numbers from 1 to $m$, where $m$ is an integer less than $n$-1 or equal to $n$-1 or greater than $n$-1,
    - to cause the output unit (30) to store the one or more representations $R_{p+q}{}^*$ and/or to output them and/or to transmit them to a separate computer system.

13. Computer program product comprising a computer program (40) that can be loaded into a working memory (22) of a computer system (1), where it causes the computer system (1) to execute the following steps:

- receiving a representation $R_p$ of an examination region of an examination object, where the representation $R_p$ represents the examination region without contrast agent or after administration of an amount $a_p$ of a contrast agent, where $p$ is an integer less than $n$, where $n$ is an integer greater than 2, where the examination object is preferably a human and the examination region is preferably part of the human,
- feeding the representation $R_p$ to a trained machine-learning model (M), where the machine-learning model (M) has been trained on the basis of training data to generate, starting from a first representation $^TR_1$, a number $n$-1 of representations $^TR_2^*$ to $^TR_n^*$ one after the other, where the first representation $^TR_1$ represents the examination region without contrast agent or after administration of a first amount $a_1$ of the contrast agent and each generated representation $^TR_j^*$ represents the examination region after an amount $a_j$ of the contrast agent, where $j$ is an index passing through integers from 2 to $n$, where the amounts $a_1$ to $a_n$ form a sequence of preferably increasing amounts, where the representation $^TR_2^*$ is generated at least partly on the basis of the representation $^TR_1$ and each further representation $^TR_k^*$ is generated at least partly on the basis of the respective previously generated representation $^TR_{k-1}^*$, where $k$ is an index passing through integers from 3 to $n$,
- receiving one or more representations $R_{p+q}^*$ of the examination region from the machine-learning model (M), where each of the one or more representations $R_{p+q}^*$ represents the examination region after administration of the amount $a_{p+q}$ of the contrast agent, where $q$ is an index passing through the numbers from 1 to $m$, where m is an integer less than $n$-1 or equal to $n$-1 or greater than $n$-1,
- outputting and/or storing and/or transmitting the one or more representations $R_{p+q}^*$.

14. Use of a contrast agent in a radiological examination method, wherein the radiological examination method comprises the following steps:

- receiving a representation $R_p$ of an examination region of an examination object, where the representation $R_p$ represents the examination region without contrast agent or after administration of an amount $a_p$ of the contrast agent, where $p$ is an integer less than $n$, where $n$ is an integer greater than 2,
- feeding the representation $R_p$ to a trained machine-learning model (M), where the machine-learning model (M) has been trained on the basis of training data to generate, starting from a first representation $^TR_1$, a number $n$-1 of representations $^TR_2^*$ to $^TR_n^*$ one after the other, where the first representation $^TR_1$ represents the examination region without contrast agent or after administration of a first amount $a_1$ of the contrast agent and each generated representation $^TR_j^*$ represents the examination region after administration of an amount $a_j$ of the contrast agent, where $j$ is an index passing through integers from 2 to $n$, where the amounts $a_1$ to $a_n$ form a sequence of preferably increasing amounts, where the representation $^TR_2^*$ is generated at least partly on the basis of the representation $^TR_1$ and each further representation $^TR_k^*$ is generated at least partly on the basis of the respective previously generated representation $^TR_{k-1}^*$, where $k$ is an index passing through integers from 3 to $n$,
- receiving one or more representations $R_{p+q}^*$ of the examination region from the machine-learning model (M), where each of the one or more representations $R_{p+q}^*$ represents the examination region after administration of the amount $a_{p+q}$ of the contrast agent, where $q$ is an index passing through the numbers from 1 to $m$, where m is an integer less than $n$-1 or equal to $n$-1 or greater than $n$-1,
- outputting and/or storing and/or transmitting the one or more representations $R_{p+q}^*$.

15. Kit comprising a contrast agent and a computer program product, wherein the computer program product comprises a computer program (40) that can be loaded into a working memory (22) of a computer system (1), where it causes the computer system (1) to execute the following steps:

- receiving a representation $R_p$ of an examination region of an examination object, where the representation $R_p$ represents the examination region without contrast agent or after administration of an amount $a_p$ of the contrast agent, where $p$ is an integer less than $n$, where $n$ is an integer greater than 2,
- feeding the representation $R_p$ to a trained machine-learning model (M), where the machine-learning model (M) has been trained on the basis of training data to generate, starting from a first representation $^TR_1$, a number $n$-1 of representations $^TR_2^*$ to $^TR_n^*$ one after the other, where the first representation $^TR_1$ represents the examination region without contrast agent or after administration of a first amount $a_1$ of the contrast agent and each generated representation $^TR_j^*$ represents the examination region after administration of an amount $a_j$ of the contrast agent, where $j$ is an index passing through integers from 2 to $n$, where the amounts $a_1$ to $a_n$ form a sequence of preferably increasing amounts, where the representation $^TR_2{}^*$ is generated at least partly on the basis of the representation $^TR_1$ and each further representation $^TR_k{}^*$ is generated at least partly on the basis of the respective previously generated representation $^TR_{k-1}{}^*$, where $k$ is an index passing through integers from 3 to $n$,

- receiving one or more representations $R_{p+q}{}^*$ of the examination region from the machine-learning model (M), where each of the one or more representations $R_{p+q}{}^*$ represents the examination region after administration of the amount $a_{p+q}$ of the contrast agent, where $q$ is an index passing through the numbers from 1 to $m$, where $m$ is an integer less than $n$-1 or equal to $n$-1 or greater than $n$-1,

- outputting and/or storing and/or transmitting the one or more representations $R_{p+q}{}^*$.

## Revendications

1. Procédé mis en œuvre par ordinateur pour l'entraînement et l'utilisation d'un modèle (M) d'apprentissage automatique, le procédé comprenant les étapes suivantes :

   - réception de données d'entraînement,

     • les données d'entraînement comprenant, pour une pluralité d'objets d'examen, des représentations $^TR_1$ à $^TR_n$ d'une région d'examen, n étant un nombre entier qui est supérieur à deux,
     • chaque représentation $^TR_i$ représentant la région d'examen après application d'une quantité $a_i$ d'un agent de contraste, i étant un nombre entier dans la plage de 1 à n, les quantités $a_1$ à $a_n$ formant une suite de quantités de préférence croissantes,
     • chaque objet d'examen étant de préférence un être humain et la région d'examen étant de préférence une partie de l'être humain,

   - entraînement du modèle (M) d'apprentissage automatique,

     • le modèle (M) étant entraîné pour générer successivement, à partir de la représentation $^TR_1$, des représentations $^TR_2{}^*$ à $^TR_n{}^*$,
     • la génération de la représentation $^TR_2{}^*$ s'effectuant au moins proportionnellement sur la base de la représentation $^TR_1$ et la génération de chaque représentation supplémentaire $^TR_k{}^*$ s'effectuant au moins proportionnellement sur la base de la représentation $^TR_{k-1}{}^*$ respectivement générée précédemment, k étant un indice qui parcourt les nombres entiers de 3 à n, la représentation $^TR_k{}^*$ représentant la région d'examen après application de la quantité $a_k$ de l'agent de contraste et la représentation $^TR_{k-1}{}^*$ représentant la région d'examen après application de la quantité $a_{k-1}$ de l'agent de contraste,
     • les écarts entre les représentations générées $^TR_j{}^*$ et les représentations $^TR_j$ étant quantifiés au moyen d'une fonction d'erreur, j étant un indice qui parcourt les nombres de 2 à n,
     • les écarts étant réduits au minimum en modifiant les paramètres de modèle (MP) du modèle (M) d'apprentissage automatique,

   - utilisation du modèle (M) d'apprentissage automatique pour la prédiction.

2. Procédé selon la revendication 1, comprenant en outre les étapes suivantes :

   - pour chaque représentation $^TR_j{}^*$ générée : calcul d'une valeur d'erreur pour chaque paire constituée d'une représentation $^TR_j$ et des représentations générées $^TR_j{}^*$ à l'aide d'une fonction d'erreur, j étant un indice qui parcourt les nombres de 2 à n,
   - calcul d'une valeur d'erreur totale à l'aide d'une fonction d'erreur totale, la fonction d'erreur totale étant une fonction des valeurs d'erreur,
   - réduction au minimum de la valeur d'erreur totale en modifiant les paramètres de modèle (MP).

3. Procédé selon la revendication 2, la fonction d'erreur totale ayant la formule suivante :

$$\mathrm{LV} \;=\; \sum_{j=2}^{n} w_j \cdot \mathrm{LF}_j$$

LV étant la valeur d'erreur totale, $\mathrm{LF}_j$ étant les fonctions d'erreur pour le calcul des valeurs d'erreur pour les écarts entre

la représentation $^TR_j$ et la représentation générée $^TR_j{}^*$, et $w_j$ étant des facteurs de pondération, j étant un indice qui parcourt les nombres de 2 à n.

4. Procédé mis en œuvre par ordinateur pour la prédiction d'une représentation d'une région d'examen d'un objet d'examen, comprenant les étapes suivantes :

- réception d'une représentation $R_p$ de la région d'examen,

   • la représentation $R_p$ représentant la région d'examen sans agent de contraste ou après application d'une quantité $a_p$ d'un agent de contraste, p étant un nombre entier qui est inférieur à n, n étant un nombre entier supérieur à 2, l'objet d'examen étant de préférence un être humain et la région d'examen étant de préférence une partie de l'être humain,
   • acheminement de la représentation $R_p$ à un modèle (M) entraîné d'apprentissage automatique,
   • le modèle (M) d'apprentissage automatique ayant été entraîné sur la base de données d'entraînement en vue de générer successivement, à partir d'une première représentation $^TR_1$, un nombre n-1 de représentations $^TR_2{}^*$ à $^TR_n{}^*$,
   • la première représentation $^TR_1$ représentant la région d'examen sans agent de contraste ou après application d'une première quantité $a_1$ de l'agent de contraste et chaque représentation générée $^TR_j{}^*$ représentant la région d'examen après application d'une quantité $a_j$ de l'agent de contraste, j étant un indice qui parcourt les nombres entiers de 2 à n, les quantités $a_1$ à $a_n$ formant une suite de quantités de préférence croissantes,
   • la génération de la représentation $^TR_2{}^*$ s'effectuant au moins proportionnellement sur la base de la représentation $^TR_1$ et la génération de chaque représentation supplémentaire $^TR_k{}^*$ s'effectuant au moins proportionnellement sur la base de la représentation $^TR_{k-1}{}^*$ respectivement générée précédemment, k étant un indice qui parcourt les nombres entiers de 3 à n,

- réception d'une ou plusieurs représentations $R_{p+q}{}^*$ de la région d'examen en provenance du modèle (M) d'apprentissage automatique,

   • chacune de l'une ou des plusieurs représentations $R_{p+q}{}^*$ représentant la région d'examen après l'application de la quantité $a_{p+q}$ de l'agent de contraste, q étant un indice qui parcourt les nombres de 1 à m, m étant un nombre entier qui est inférieur à n-1 ou est égal à n-1 ou est supérieur à n-1,

- délivrance en sortie et/ou mémorisation et/ou communication de l'une ou des plusieurs représentations $R_{p+q}{}^*$.

5. Procédé selon la revendication 4, le procédé comprenant les étapes suivantes :

- réception de la représentation $R_p$ de la région d'examen, la représentation $R_p$ représentant la région d'examen sans agent de contraste ou après application de la quantité $a_p$ d'agent de contraste, p étant un nombre entier qui est inférieur à n,
- acheminement de la représentation $R_p$ au modèle (M) entraîné d'apprentissage automatique entraîné, le modèle (M) d'apprentissage automatique ayant été entraîné dans un procédé selon l'une des revendications 1 à 3,
- réception de l'une ou des plusieurs représentations $R_{p+q}{}^*$ de la région d'examen en provenance du modèle (M) d'apprentissage automatique, chacune de l'une ou des plusieurs représentations $R_{p+q}{}^*$ représentant la région d'examen après l'application de la quantité $a_{p+q}$ de l'agent de contraste, q étant un indice qui parcourt les nombres de 1 à m, m étant un nombre entier qui est inférieur à n-1 ou est égal à n-1 ou est supérieur à n-1,
- délivrance en sortie et/ou mémorisation et/ou communication de l'une ou des plusieurs représentations $R_{p+q}{}^*$.

6. Procédé selon l'une des revendications 1 à 5, chaque représentation $^TR_{p+q}{}^*$ et/ou $R_{p+q}{}^*$ générée par le modèle (M) d'apprentissage automatique étant de nouveau acheminée au modèle (M) entraîné d'apprentissage automatique afin de générer une représentation $^TR_{p+q+1}{}^*$ et/ou $R_{p+q+1}{}^*$.

7. Procédé selon l'une des revendications 4 à 6, m se situant dans la plage de n à n+10.

8. Procédé selon l'une des revendications 1 à 7, chaque représentation générée étant calculée selon la formule suivante :

$$M^q \ (R_1) \ = \ R_{1+q}{}^*$$

M représentant une transformation qui est appliquée à des données d'entrée du modèle (M) d'apprentissage automatique par le modèle (M) d'apprentissage automatique, $M^q$ désigne la q-ième application de la transformation, la transformation étant appliquée dans une première étape à une première représentation $R_1$, la transformation étant appliquée dans une deuxième étape au résultat de l'application effectuée dans la première étape en réacheminant le résultat dans le modèle (M) d'apprentissage automatique et en répétant le processus avec chaque résultat supplémentaire d'une transformation jusqu'à ce que la transformation ait été appliquée au total q fois, q étant un nombre entier qui peut prendre les valeurs de 1 à m, m étant un nombre entier qui est inférieur à n-1 ou est égal à n-1 ou est supérieur à n-1.

9. Procédé selon l'une des revendications 1 à 8, la région d'examen étant le foie humain ou une partie du foie humain.

10. Procédé selon l'une des revendications 1 à 9, les représentations reçues étant des images de tomodensitométrie, des images IRM ou des images échographiques.

11. Procédé selon l'une des revendications 1 à 9, les représentations reçues étant des images de tomodensitométrie et l'agent de contraste étant un agent de contraste MR.

12. Système informatique (1) comprenant

   • une unité d'entrée (10),
   • une unité de commande et de calcul (20), et
   • une unité de sortie (30),

   l'unité de commande et de calcul (20) étant configurée pour

   - amener l'unité d'entrée (10) à recevoir une représentation $R_p$ d'une région d'examen d'un objet d'examen, la représentation $R_p$ représentant la région d'examen sans agent de contraste ou après application d'une quantité $a_p$ d'un agent de contraste, p étant un nombre entier qui est inférieur à n, n étant un nombre entier supérieur à 2, l'objet d'examen étant de préférence un être humain et la région d'examen étant de préférence une partie de l'être humain,
   - acheminer la représentation $R_p$ à un modèle (M) entraîné d'apprentissage automatique, le modèle (M) d'apprentissage automatique ayant été entraîné sur la base de données d'entraînement afin de générer successivement, à partir d'une première représentation $^TR_1$, un nombre n-1 de représentations $^TR_2{}^*$ à $^TR_n{}^*$, la première représentation $^TR_1$ représentant la région d'examen sans agent de contraste ou après l'application d'une première quantité $a_1$ de l'agent de contraste et chaque représentation générée $^TR_j{}^*$ représentant la région d'examen après l'application d'une quantité $a_j$ de l'agent de contraste, j étant un indice qui parcourt les nombres entiers de 2 à n, les quantités $a_1$ à $a_n$ formant une suite de quantités de préférence croissantes, la génération de la représentation $^TR_2{}^*$ s'effectuant au moins proportionnellement sur la base de la représentation $^TR_1$ et la génération de chaque représentation supplémentaire $^TR_k{}^*$ s'effectuant au moins proportionnellement sur la base de la représentation $^TR_{k-1}{}^*$ respectivement générée précédemment, k étant un indice qui parcourt les nombres entiers de 3 à n,
   - recevoir, en provenance du modèle (M) d'apprentissage automatique, une ou plusieurs représentations $R_{p+q}{}^*$ de la région d'examen, chacune de l'une ou des plusieurs représentations $R_{p+q}{}^*$ représentant la région d'examen après l'application de la quantité $a_{p+q}$ de l'agent de contraste, q étant un indice qui parcourt les nombres de 1 à m, m étant un nombre entier qui est inférieur à n-1 ou est égal à n-1 ou est supérieur à n-1,
   - amener l'unité de sortie (30) à mémoriser et/ou à délivrer en sortie et/ou à communiquer à un système informatique séparé l'une ou les plusieurs représentations $R_{p+q}{}^*$.

13. Produit de programme informatique comprenant un programme informatique (40) qui est apte à être chargé dans une mémoire de travail (22) d'un système informatique (1) et qui, dans celui-ci, amène le système informatique (1) à exécuter les étapes suivantes :

   - réception d'une représentation $R_p$ d'une région d'examen d'un objet d'examen, la représentation $R_p$ représentant la région d'examen sans agent de contraste ou après application d'une quantité $a_p$ d'un agent de contraste, p étant un nombre entier qui est inférieur à n, n étant un nombre entier supérieur à 2, l'objet d'examen étant de préférence un être humain et la région d'examen étant de préférence une partie de l'être humain,
- acheminement de la représentation $R_p$ à un modèle (M) entraîné d'apprentissage automatique, le modèle (M) d'apprentissage automatique ayant été entraîné sur la base de données d'entraînement afin de générer successivement, à partir d'une première représentation $^TR_1$, un nombre n-1 de représentations $^TR_2^*$ à $^TR_n^*$, la première représentation $^TR_1$ représentant la région d'examen sans agent de contraste ou après l'application d'une première quantité $a_1$ de l'agent de contraste et chaque représentation générée $^TR_j^*$ représentant la région d'examen après une quantité $a_j$ de l'agent de contraste, j étant un indice qui parcourt les nombres entiers de 2 à n, les quantités $a_1$ à $a_n$ formant une suite de quantités de préférence croissantes, la génération de la représentation $^TR_2^*$ s'effectuant au moins proportionnellement sur la base de la représentation $^TR_1$ et la génération de chaque représentation supplémentaire $^TR_k^*$ s'effectuant au moins proportionnellement sur la base de la représentation $^TR_{k-1}^*$ respectivement générée précédemment, k étant un indice qui parcourt les nombres entiers de 3 à n,
- réception d'une ou plusieurs représentations $R_{p+q}^*$ de la région d'examen en provenance du modèle (M) d'apprentissage automatique, chacune de l'une ou des plusieurs représentations $R_{p+q}^*$ représentant la région d'examen après l'application de la quantité $a_{p+q}$ de l'agent de contraste, q étant un indice qui parcourt les nombres de 1 à m, m étant un nombre entier qui est inférieur à n-1 ou est égal à n-1 ou est supérieur à n-1,
- délivrance en sortie et/ou mémorisation et/ou communication de l'une ou des plusieurs représentations $R_{p+q}^*$.

14. Utilisation d'un agent de contraste dans un procédé d'examen radiologique, le procédé d'examen radiologique comprenant les étapes suivantes :

- réception d'une représentation $R_p$ d'une région d'examen d'un objet d'examen, la représentation $R_p$ représentant la région d'examen sans agent de contraste ou après application d'une quantité $a_p$ d'un agent de contraste, p étant un nombre entier qui est inférieur à n, n étant un nombre entier supérieur à 2,
- acheminement de la représentation $R_p$ à un modèle (M) entraîné d'apprentissage automatique, le modèle (M) d'apprentissage automatique ayant été entraîné sur la base de données d'entraînement afin de générer successivement, à partir d'une première représentation $^TR_1$, un nombre n-1 de représentations $^TR_2^*$ à $^TR_n^*$, la première représentation $^TR_1$ représentant la région d'examen sans agent de contraste ou après l'application d'une première quantité $a_1$ de l'agent de contraste et chaque représentation générée $^TR_j^*$ représentant la région d'examen après l'application d'une quantité $a_j$ de l'agent de contraste, j étant un indice qui parcourt les nombres entiers de 2 à n, les quantités $a_1$ à $a_n$ formant une suite de quantités de préférence croissantes, la génération de la représentation $^TR_2^*$ s'effectuant au moins proportionnellement sur la base de la représentation $^TR_1$ et la génération de chaque représentation supplémentaire $^TR_k^*$ s'effectuant au moins proportionnellement sur la base de la représentation $^TR_{k-1}^*$ respectivement générée précédemment, k étant un indice qui parcourt les nombres entiers de 3 à n,
- réception d'une ou plusieurs représentations $R_{p+q}^*$ de la région d'examen en provenance du modèle (M) d'apprentissage automatique, chacune de l'une ou des plusieurs représentations $R_{p+q}^*$ représentant la région d'examen après l'application de la quantité $a_{p+q}$ de l'agent de contraste, q étant un indice qui parcourt les nombres de 1 à m, m étant un nombre entier qui est inférieur à n-1 ou est égal à n-1 ou est supérieur à n-1,
- délivrance en sortie et/ou mémorisation et/ou communication de l'une ou des plusieurs représentations $R_{p+q}^*$.

15. Kit comprenant un agent de contraste et un produit de programme informatique, le produit de programme informatique comprenant un programme informatique (40) qui peut être chargé dans une mémoire de travail (22) d'un système informatique (1) et qui y amène le système informatique (1) à exécuter les étapes suivantes :

- réception d'une représentation $R_p$ d'une région d'examen d'un objet d'examen, la représentation $R_p$ représentant la région d'examen sans agent de contraste ou après application d'une quantité $a_p$ d'un agent de contraste, p étant un nombre entier qui est inférieur à n, n étant un nombre entier supérieur à 2,
- acheminement de la représentation $R_p$ à un modèle (M) entraîné d'apprentissage automatique, le modèle (M) d'apprentissage automatique ayant été entraîné sur la base de données d'entraînement afin de générer successivement, à partir d'une première représentation $^TR_1$, un nombre n-1 de représentations $^TR_2^*$ à $^TR_n^*$, la première représentation $^TR_1$ représentant la région d'examen sans agent de contraste ou après l'application d'une première quantité $a_1$ de l'agent de contraste et chaque représentation générée $^TR_j^*$ représentant la région d'examen après l'application d'une quantité $a_j$ de l'agent de contraste, j étant un indice qui parcourt les nombres entiers de 2 à n, les quantités $a_1$ à $a_n$ formant une suite de quantités de préférence croissantes, la génération de la représentation $^TR_2^*$ s'effectuant au moins proportionnellement sur la base de la représentation $^TR_1$ et la génération de chaque représentation supplémentaire $^TR_k^*$ s'effectuant au moins proportionnellement sur la base de la représentation $^TR_{k-1}^*$ respectivement générée précédemment, k étant un indice qui parcourt les nombres entiers de 3 à n,

- réception d'une ou plusieurs représentations $R_{p+q}*$ de la région d'examen en provenance du modèle (M) d'apprentissage automatique, chacune de l'une ou des plusieurs représentations $R_{p+q}*$ représentant la région d'examen après l'application de la quantité $a_{p+q}$ de l'agent de contraste, q étant un indice qui parcourt les nombres de 1 à m, m étant un nombre entier qui est inférieur à n-1 ou est égal à n-1 ou est supérieur à n-1,

- délivrance en sortie et/ou mémorisation et/ou communication de l'une ou des plusieurs représentations $R_{p+q}*$.

Fig. 1

Fig. 2

Fig. 3

Fig. 4

Fig. 5

$(q-1)\,x$

$R_p$
$(a_p)$

M
(MP)

S

$R_{p+q}$
$(a_{p+q})$

**Fig. 6**

(10)          (20)          (30)

(1)

**Fig. 7**

(1)

(31)

(11)

(32)

(21)

(12)

(22)

(33)

(40)

**Fig. 8**

(110)

(120)

(130)

(100)

Fig. 9

(210)

(220)

(230)

(240)

(200)

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2021044153 A1
- US 2020294288 A1
- EP 3739522 A1
- WO 2019074938 A1
- US 6039931 A

**In der Beschreibung aufgeführte Nicht-Patentliteratur**

- **Z.B. A. S. L. JASCINTH et al.** Contrast Agents in computed tomography: A Review. *Journal of Applied Dental and Medical Sciences*, 2016, vol. 2 (2), 143-149
- **H. LUSIC et al.** X-ray-Computed Tomography Contrast Agents. *Chem. Rev.*, 2013, vol. 113 (3), 1641-1666, https://www.radiology.wisc.edu/wp-content/uploads/2017/10/contrast-agents-tutorial.pdf
- **M. R. NOUGH et al.** Radiographic and magnetic resonances contrast agents: Essentials and tips for safe practices. *World J Radiol.*, 28 September 2017, vol. 9 (9), 339-349
- **L. C. ABONYI et al.** Intravascular Contrast Media in Radiography: Historical Development & Review of Risk Factors for Adverse Reactions. *South American Journal of Clinical Research*, 2016, vol. 3 (1), 1-10
- *ACR Manual on Contrast Media*, 2020, ISBN 978-1-55903-012-0
- **A. IGNEE et al.** Ultrasound contrast agents. *Endosc Ultrasound.*, November 2016, vol. 5 (6), 355-362
- **R. MECHREZ et al.** The Contextual Loss for Image Transformation with Non-Aligned Data. *arXiv:1803.02077v4*, 2018
- **H. ZHAO et al.** Loss Functions for Image Restoration with Neural Networks. *arXiv:1511.08861v3*, 2018
- U-net: Convolutional networks for biomedical image segmentation. **O. RONNEBERGER et al.** International Conference on Medical image computing and computer-assisted intervention. Springer, 2015, 234-241
- **M.-Y. LIU et al.** Generative Adversarial Networks for Image and Video Synthesis: Algorithms and Applications. *arXiv:2008.02793*
- **J. HENRY et al.** *Pix2Pix GAN for Image-to-Image Translation*
- **Y. GAO et al.** *Fully convolutional structured LSTM networks for joint 4D medical image segmentation*
- **D. KARIMI et al.** Convolution-Free Medical Image Segmentation using Transformers. *arXiv:2102.13645 [eess.IV*
- **L.P. SMITS et al.** Evaluation of ultrasmall superparamagnetic iron-oxide (USPIO) enhanced MRI with ferumoxytol to quantify arterial wall inflammation. *Atherosclerosis*, 2017, vol. 263, 211-218